(19) Europäisches Patentamt
European Patent Office
Office européen des brevets

(11) EP 4 741 489 A1

(12) EUROPEAN PATENT APPLICATION
published in accordance with Art. 153(4) EPC

(43) Date of publication:
**13.05.2026 Bulletin 2026/20**

(21) Application number: **24843137.1**

(22) Date of filing: **16.07.2024**

(51) International Patent Classification (IPC):
***C12M 3/00*** *(2006.01)* ***C12N 5/071*** *(2010.01)*

(52) Cooperative Patent Classification (CPC):
**C12M 3/00; C12N 5/06**

(86) International application number:
**PCT/JP2024/025525**

(87) International publication number:
**WO 2025/018347 (23.01.2025 Gazette 2025/04)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **20.07.2023 JP 2023118581**

(71) Applicant: **Canon Kabushiki Kaisha**
**Tokyo 146-8501 (JP)**

(72) Inventors:
- **TAKEMURA, Kenjiro**
  **Yokohama-shi, Kanagawa 223-8522 (JP)**
- **KURASHINA, Yuta**
  **Yokohama-shi, Kanagawa 223-8522 (JP)**
- **IMASHIRO, Chikahiro**
  **Yokohama-shi, Kanagawa 223-8522 (JP)**
- **TSUBAKI, Keiichiro**
  **Tokyo 146-8501 (JP)**
- **FURUI ,Takaaki**
  **Tokyo 146-8501 (JP)**
- **NAKAMOTO, Atsushi**
  **Tokyo 146-8501 (JP)**
- **NOGUCHI, Kazunori**
  **Tokyo 146-8501 (JP)**

(74) Representative: **WESER & Kollegen**
**Patentanwälte PartmbB**
**Radeckestraße 43**
**81245 München (DE)**

# (54) CELL DETACHMENT METHOD AND CELL DETACHMENT DEVICE

(57) Provided are a cell detachment method and a cell detachment device that enable vibration design suitable for target cells and detachment of adherent cells at a high detachment rate. The cell detachment method of detaching cells adhering to a culture surface of a culture substrate from the culture surface includes a detachment step of detaching the cells from the culture surface by vibrating a vibrating body under a state in which a first acoustic transmission medium and a second acoustic transmission medium are arranged between the culture substrate and the vibrating body, and under a state in which at least one of the following (a) or (b) is satisfied: (a) the culture substrate is in contact with the first acoustic transmission medium and the second acoustic transmission medium; or (b) the vibrating body is in contact with the first acoustic transmission medium and the second acoustic transmission medium. In the cell detachment method, the first acoustic transmission medium and the second acoustic transmission medium are different from each other, the first acoustic transmission medium being a solid at room temperature, the second acoustic transmission medium being a liquid or a solid with flowability at room temperature.

FIG. 1A

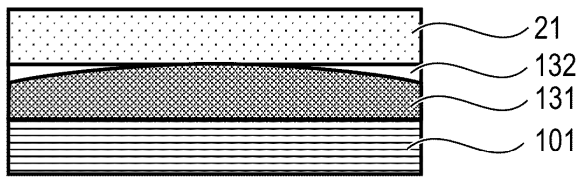


EP 4 741 489 A1

## Description

[Technical Field]

The present disclosure relates to a cell detachment method and a cell detachment device.

[Background Art]

In recent years, cell culturing in which cells to be used in regenerative medicine and cells to be used in the manufacture of biopharmaceuticals and the like are cultured has been actively performed. Cell culturing is roughly classified into adhesion culture and suspension culture, and of those, adhesion culture is a culturing method suitable for various cell types and is widely used. However, at the time of collecting cells, it is required to perform enzyme treatment to detach the cells, and in some treatment methods, the cells may be damaged. Thus, cell detachment through enzyme treatment is also a cause of variation in cell quality. For that reason, studies have been made on detachment conditions for collecting cells at a high detachment rate without damaging the cells.

In Patent Literature 1, there is proposed a patterning device that arranges cells at specific positions through a medium that transmits acoustic radiation pressure to a culture substrate. However, a liquid such as water is used as the acoustic transmission medium, and hence there is a risk of contamination due to the liquid of the vibration transmission medium entering the inside of the culture substrate when vibration is applied, depending on the amount of the acoustic transmission medium used. In addition, when silicone rubber is used as the acoustic transmission medium, vibration generated by a vibration generation source may not be successfully transmitted to the culture substrate, or the vibration may change.

[Citation List]

[Patent Literature]

PTL 1: Japanese Patent Laid-Open No. 2018-42534

[Summary of Invention]

[Technical Problem]

The present disclosure has been made to address the above-mentioned problems, and is directed to providing a cell detachment method and a cell detachment device that enable vibration design suitable for target cells and detachment of adherent cells at a high detachment rate.

[Solution to Problem]

According to the present disclosure, there is provided a cell detachment method of detaching cells adhering to a culture surface of a culture substrate from the culture surface, the cell detachment method including a detachment step of detaching the cells from the culture surface by vibrating a vibrating body under a state in which a first acoustic transmission medium and a second acoustic transmission medium are arranged between the culture substrate and the vibrating body, and under a state in which at least one of the following (a) or (b) is satisfied:

(a) the culture substrate is in contact with the first acoustic transmission medium and the second acoustic transmission medium; or
(b) the vibrating body is in contact with the first acoustic transmission medium and the second acoustic transmission medium,

wherein the first acoustic transmission medium and the second acoustic transmission medium are different from each other, the first acoustic transmission medium being a solid at room temperature, the second acoustic transmission medium being a liquid or a solid with flowability at room temperature.

According to the present disclosure, the following cell detachment device is also provided.

A cell detachment device for detaching cells from a culture surface of a culture substrate by transmitting vibration generated by a vibrating body to the cells provided on the culture surface, the cell detachment device including:

a vibrating body;

a first acoustic transmission medium; and
a culture substrate placement portion, in the stated order, and
the cell detachment device further including supply unit for supplying a second acoustic transmission medium,
wherein the supply unit is configured to supply the second acoustic transmission medium so that at least one of the following (a) or (b) is satisfied:

(a) the culture substrate is in contact with the first acoustic transmission medium and the second acoustic transmission medium; or
(b) the vibrating body is in contact with the first acoustic transmission medium and the second acoustic transmission medium.

[Advantageous Effects of Invention]

According to the present disclosure, it is possible to provide the cell detachment method that enables vibration design suitable for target cells and detachment of adherent cells at a high detachment rate.

[Brief Description of Drawings]

[Fig. 1A]
Fig. 1A is a schematic cross-sectional view for illustrating an example of a configuration of a cell detachment device according to a first embodiment of the present disclosure.
[Fig. 1B]
Fig. 1B is a schematic cross-sectional view for illustrating an example of the configuration of the cell detachment device according to the first embodiment of the present disclosure.
[Fig. 1C]
Fig. 1C is a schematic cross-sectional view for illustrating an example of the configuration of the cell detachment device according to the first embodiment of the present disclosure.
[Fig. 2A]
Fig. 2A is a schematic cross-sectional view for illustrating an example of a configuration of a cell detachment device according to a second embodiment of the present disclosure.
[Fig. 2B]
Fig. 2B is a schematic cross-sectional view for illustrating an example of the configuration of the cell detachment device according to the second embodiment of the present disclosure.
[Fig. 2C]
Fig. 2C is a schematic cross-sectional view for illustrating an example of the configuration of the cell detachment device according to the second embodiment of the present disclosure.
[Fig. 3A]
Fig. 3A is a schematic cross-sectional view for illustrating an example of a configuration of a cell detachment device according to a third embodiment of the present disclosure.
[Fig. 3B]
Fig. 3B is a schematic cross-sectional view for illustrating an example of the configuration of the cell detachment device according to the third embodiment of the present disclosure.
[Fig. 3C]
Fig. 3C is a schematic cross-sectional view for illustrating an example of the configuration of the cell detachment device according to the third embodiment of the present disclosure.
[Fig. 4A]
Fig. 4A is a schematic cross-sectional view for illustrating an example of a configuration of a cell detachment device according to a fourth embodiment of the present disclosure.
[Fig. 4B]
Fig. 4B is a schematic cross-sectional view for illustrating an example of the configuration of the cell detachment device according to the fourth embodiment of the present disclosure.
[Fig. 4C]
Fig. 4C is a schematic cross-sectional view for illustrating an example of the configuration of the cell detachment device according to the fourth embodiment of the present disclosure.
[Fig. 5A]
Fig. 5A is a flow chart for illustrating steps of a cell detachment method according to the present disclosure.

[Fig. 5B]
Fig. 5B is a flow chart for illustrating a specific example of the steps of the cell detachment method according to the present disclosure.
[Fig. 6A]
Fig. 6A is a view of an example of the device according to the present disclosure.
[Fig. 6B]
Fig. 6B is a view of an example of the device according to the present disclosure.
[Fig. 6C]
Fig. 6C is a view of an example of the device according to the present disclosure including an information processing device.
[Fig. 7A]
Fig. 7A is a view for illustrating an example of an arrangement step of the method according to the present disclosure.
[Fig. 7B]
Fig. 7B is a view for illustrating an example of the arrangement step of the method according to the present disclosure.
[Fig. 7C]
Fig. 7C is a view for illustrating an example of a pressing step of the method according to the present disclosure.
[Fig. 7D]
Fig. 7D is a view for illustrating an example of a detachment step of the method according to the present disclosure.
[Fig. 8]
Fig. 8 is a schematic view for illustrating an example of a culture substrate in the present disclosure.
[Fig. 9A]
Fig. 9A is a schematic view for illustrating an example of a first acoustic transmission medium in the present disclosure.
[Fig. 9B]
Fig. 9B is a schematic view for illustrating an example of the first acoustic transmission medium in the present disclosure.
[Fig. 9C]
Fig. 9C is a schematic view for illustrating an example of the first acoustic transmission medium in the present disclosure.
[Fig. 9D]
Fig. 9D is a schematic view for illustrating an example of the first acoustic transmission medium in the present disclosure.
[Fig. 9E]
Fig. 9E is a schematic view for illustrating an example of the first acoustic transmission medium in the present disclosure.
[Fig. 9F]
Fig. 9F is a cross-sectional view and a top view for illustrating an example of the first acoustic transmission medium in the present disclosure.
[Fig. 10]
Fig. 10 is a schematic view for illustrating an example of a piezoelectric body in the present disclosure.
[Fig. 11]
Fig. 11 is a view for illustrating a groove portion of the first acoustic transmission medium in the present disclosure.
[Fig. 12]
Fig. 12 is a table for showing results obtained by summarizing respective configurations and evaluation results of Examples and Comparative Examples of the present disclosure.

[Description of Embodiments]

A cell detachment method according to the present disclosure is described below by giving Examples, but the present disclosure is not limited to the following Examples.

(Basic Configuration)

The cell detachment method according to the present disclosure is a cell detachment method of detaching cells adhering to a culture surface of a culture substrate from the culture surface,

the cell detachment method including a detachment step of detaching the cells from the culture surface by vibrating a vibrating body under a state in which a first acoustic transmission medium and a second acoustic transmission medium are arranged between the culture substrate and the vibrating body, and under a state in which at least one of the following (a) or (b) is satisfied:

(a) the culture substrate is in contact with the first acoustic transmission medium and the second acoustic transmission medium; or
(b) the vibrating body is in contact with the first acoustic transmission medium and the second acoustic transmission medium,

wherein the first acoustic transmission medium and the second acoustic transmission medium are different from each other, the first acoustic transmission medium being a solid at room temperature, the second acoustic transmission medium being a liquid or a solid with flowability at room temperature. The state in which at least one of the (a) or the (b) is satisfied is achieved by the above-mentioned cell detachment method according to the present disclosure, thereby obtaining an example of the cell detachment method according to the present disclosure as illustrated in Fig. 5A. In addition, conceptual views of respective steps are illustrated in Fig. 7A to Fig. 7D.

Fig. 7A and Fig. 7B are views of a step of arranging the first acoustic transmission medium and the second acoustic transmission medium. The first acoustic transmission medium and the second acoustic transmission medium may be arranged in advance, and this step is not an essential step for the cell detachment method according to the present disclosure.

In Fig. 7A, the first acoustic transmission medium is arranged on the vibrating body, and the second acoustic transmission medium is further applied and arranged on the first acoustic transmission medium. In Fig. 7B, the culture substrate is arranged onto the arranged acoustic transmission media. Fig. 7C is a view of a pressing step in which pressure is applied from above the culture substrate. The pressing step is not essential for the detachment method according to the present disclosure. Fig. 7D is a view of the detachment step. In Fig. 7D, while pressure is applied from above the culture substrate, the vibrating body is driven to apply ultrasonic vibration and detach the cells. With the pressing step, it is easy to bring the first acoustic transmission medium and the second acoustic transmission medium into contact with the culture substrate and the vibrating body, and the first acoustic transmission medium and the second acoustic transmission medium can also transmit vibration under a state of being in contact with the culture substrate and the vibrating body during the detachment step. Herein, the phrase "state in which the first acoustic transmission medium and the second acoustic transmission medium are in contact with the culture substrate and the vibrating body" means that the first acoustic transmission medium and the second acoustic transmission medium are in sufficient contact with the culture substrate and the vibrating body so as to sufficiently transmit the vibration of the vibrating body to the culture substrate through the first acoustic transmission medium and the second acoustic transmission medium, but also means that an extremely minute gap or an extremely small amount of substance may be interposed between the first acoustic transmission medium or the second acoustic transmission medium and the culture substrate or the vibrating body as long as the problems of the present invention can be solved. For example, even when water of about several molecules is interposed between the first acoustic transmission medium and the culture substrate, it can be considered that the first acoustic transmission medium and the culture substrate are in contact with each other.

The culture substrate varies greatly among model numbers of different manufacturers and among production lots, and when both the culture substrate and the vibrating body are to be bonded to the first acoustic transmission medium, it is difficult to eliminate alignment mismatches caused by subtle differences in shape. Thus, it is difficult to entirely bond both the culture substrate and the vibrating body to the first acoustic transmission medium that exhibits small variation in shape. Further, a different culture substrate is used for each culture, and hence bonding the contact surface between the culture substrate and the first acoustic transmission medium requires work for each use, thereby complicating the procedure. In particular, when pressure is applied by selectively pressing a central portion of a cell container after being placed on the vibrating body and subjected to cell culturing, the cells may be damaged, and hence it is difficult to bring the culture substrate and the first acoustic transmission medium into close contact with each other. In the same manner, it is difficult to bring the vibrating body and the first acoustic transmission medium into close contact with each other even when the culture substrate is brought into contact with the first acoustic transmission medium and then the first acoustic transmission medium is placed on the vibrating body. The inventors of the present application have consequently arrived at the problem that it is practically difficult to improve alignment between the culture substrate and the first acoustic transmission medium and between the vibrating body and the first acoustic transmission medium.

As a result of diligent study, the inventors of the present application have found that it is important for the first acoustic transmission medium and the second acoustic transmission medium to be in contact with at least one of the culture substrate or the vibrating body. In the cell detachment method according to the present disclosure, to transmit the vibration of the vibrating body to the culture substrate, the first acoustic transmission medium and the second acoustic transmission medium are provided therebetween. The first acoustic transmission medium and the second acoustic transmission medium can complement each other to be brought into close contact with the vibrating body or the culture substrate, and hence this configuration is inferred to enable efficient vibration transmission.

The vibrating body serving as a vibration generation portion includes a piezoelectric body and a vibrating plate, and has a vibration mechanism for generating flexural vibration in which the vibration is amplified by a combination of hardness of the piezoelectric body that expands and contracts itself and hardness of the vibrating plate bonded thereto. In the present disclosure, resonance due to structural factors of the vibrating body is used, and large vibration can be obtained at a resonance frequency.

(Acoustic Transmission Medium)

In the cell detachment method according to the present disclosure, an acoustic transmission medium is used for alignment between the vibrating body that generates vibration and the culture substrate that is an object to which the vibration is to be transmitted. For the alignment, not only physical alignment of acoustic impedance or the like but also mechanical alignment of adhesion or the like is important. It is preferred to provide a configuration capable of solving the problems of the present invention by achieving the mechanical alignment and the physical alignment between the first acoustic transmission medium or the second acoustic transmission medium and the culture substrate or the vibrating body due to the first acoustic transmission medium and the second acoustic transmission medium that are in contact with at least one of the culture substrate or the vibrating body. In this embodiment, the acoustic transmission medium can also be rephrased as an acoustic transmission material.

The physical alignment between the vibrating body and the culture substrate can be defined by a physical quantity called "acoustic impedance."

Specifically, an acoustic impedance Z of the acoustic transmission medium is defined by:

Z=(density of the acoustic transmission medium) ×(sound speed inside the acoustic transmission medium).

Further, when sound waves reach a boundary between two substances, a transmittance of the sound waves is determined by:

$$\text{Transmittance}= (4\times Z1\times Z2)/(Z1+Z2)^{\wedge}2,$$

where Z1 and Z2 are acoustic impedances of the first and second substances that transmit the sound waves.

For example, the transmittance between water and air, and the transmittance between silicone rubber and air is almost zero by substituting the impedances of water and air into the above formula:

$$(\text{Acoustic impedance Z1 of water})=1.5\times 10^{\wedge}6\ [\text{kg/m}^{\wedge}2/\text{s}]$$

$$(\text{Acoustic impedance Z2 of air})=4.08\times 10^{\wedge}2\ [\text{kg/m}^{\wedge}2/\text{s}]$$

$$(\text{Acoustic impedance Z3 of silicone rubber})=1.0\times 10^{\wedge}6\ [\text{kg/m}^{\wedge}2/\text{s}]$$

That is, the transmittances of the sound waves between water and air and between silicone rubber and air are almost zero, resulting in total reflection.

An acoustic impedance of polystyrene, which is often used as a material for culture substrates, is Z4=2.4×10^6 [kg/m^2/s]. Thus, in regard to the physical alignment between the vibrating body and the culture substrate in the acoustic transmission medium in the present disclosure, it is preferred to use materials having an acoustic impedance close to the above-mentioned value for the first acoustic transmission medium and the second acoustic transmission medium.

In regard to the mechanical alignment between the vibrating body and the culture substrate, a state of being in close contact with each other through a solid is preferred. In particular, in the present disclosure, in order to form a vibration waveform of the vibrating body on the cell culture surface of the culture substrate, such mechanical alignment as to allow close contact over a wide area is important.

It is assumed that a commercially available dish or the like having a projecting portion is used as the culture substrate and placed directly on the vibrating body. Under this state, even when a low-molecular-weight material such as water or glycerin, which is easily deformed by a low load, is used as the acoustic transmission medium, the projecting portion of the culture substrate comes into direct contact with the vibrating body, thereby generating a plurality of vibration transmission paths. As a result, the generated vibration becomes complex, and hence it becomes difficult to control the vibration. In the cell detachment method according to the present disclosure, active vibration control is performed. That is, the vibration transmission path is kept constant by bringing the vibrating body and the culture substrate into close contact with each other through intermediation of a solid, and the vibration control is thereby facilitated.

When the vibrating body and the culture substrate are mechanically aligned through use of the acoustic transmission medium in the present disclosure, it is preferred to use, as a solid material, an elastic material such as rubber or gel, which is a polymer material. Specific examples thereof include silicone resin, urethane resin, diallyl phthalate resin, unsaturated polyester, epoxy resin, phenol resin, urea resin, melamine resin, and natural rubber. Those materials may be porous resin materials. Of those, silicone rubber is preferred because of having high transmission efficiency due to a physical property of low attenuation in a frequency band of a driving frequency (vibration frequency) and also because of being able to suppress heat generation of the acoustic transmission medium itself.

(First Acoustic Transmission Medium)

The first acoustic transmission medium is a solid at room temperature. The term "room temperature" as used herein refers to 20°C or more and 30°C or less, preferably 25°C.

It is preferred that the solid have elasticity. Examples of the elastic solid material can include rubber and gel, which are polymer materials.

Examples of the rubber as polymer materials include rubber containing at least one type of resin selected from the group consisting of silicone resin, urethane resin, diallyl phthalate resin, unsaturated polyester, epoxy resin, phenol resin, urea resin, and melamine resin; and natural rubber. More specific examples include silicone rubber, urethane rubber, butyl rubber, nitrile rubber, butadiene rubber, and latex rubber.

Examples of gel as polymer materials include hydrogels and polymer gels. Examples of the hydrogels include those obtained by combining a hydrophilic polymer material such as polyvinyl alcohol or polyacrylic acid with a cross-linking agent. Examples of cross-linking agents include inorganic agents such as borax or a layered clay mineral (clay), and organic agents such as polyfunctional polymer materials, for example, dendritic polymers. Examples of polymer gels include those obtained by combining a polymer material such as acrylic resin or urethane resin with a polyfunctional compound serving as a corresponding cross-linking agent.

It is preferred to use, as the first acoustic transmission medium, an acoustic transmission medium for which a tanδ obtained by dynamic viscoelasticity measurement at room temperature when a vibration having a frequency of 1 Hz is applied to the first acoustic transmission medium is less than 1.0.

The first acoustic transmission medium preferably includes at least one type selected from the group consisting of silicone rubber, natural rubber, urethane gel, and gel materials, and of those, silicone rubber is preferred to be included. Silicone rubber has high transmission efficiency because of its low attenuation within the frequency range of the driving (vibration) frequency, and can also suppress heat generation of the acoustic transmission medium itself.

In addition, for the purpose of facilitating removal, surface treatment such as a fluorinated coating may be applied to a surface of the first acoustic transmission medium facing the culture substrate or the vibrating body.

Further, the acoustic transmission medium is preferably transparent to visible light range in order to directly observe a cell detachment state.

Various additives may be added to the first acoustic transmission medium for the purpose of controlling physical property values such as viscosity, affinity with the second acoustic transmission medium, acoustic impedance with the second acoustic transmission medium, storage stability, and antibacterial activity. Affinity refers to properties such as a contact angle and wettability between the first and second acoustic transmission media.

(Second Acoustic Transmission Medium)

The second acoustic transmission medium is a material different from that of the first acoustic transmission medium, and is a liquid or a solid with flowability at room temperature. The term " flowability" refers to a capability to flow or move under gravity when tilted. Examples of the solid with flowability include a solid for which a tanδ obtained by dynamic viscoelasticity measurement at room temperature when a vibration having a frequency of 0.1 Hz is applied to the solid material is 1 or more.

The phrase " liquid at room temperature" refers to a liquid state at 20°C to 30°C. The material preferably has one of the following: a low intrinsic viscosity [η] at 25°C; or a low complex viscosity η* obtained by dynamic viscoelasticity measurement, or a low viscosity η measured by changing a shear rate by a B-type viscometer or a rheometer, at the room temperature of 25°C when a vibration having a frequency of 1 Hz is applied.

When the second acoustic transmission medium is a solid with flowability, it is preferred that the second acoustic transmission medium have a tanδ greater than that of the first acoustic transmission medium, that is, a solid material having a tanδ greater than that of the first acoustic transmission medium, or a liquid material, is preferred for the second acoustic transmission medium. Specifically, it is preferred to satisfy $\tan\delta_1 < \tan\delta_2$, where $\tan\delta_1$ and $\tan\delta_2$ are the tanδ of the first acoustic transmission medium and the tanδ of the second acoustic transmission medium, respectively, obtained by dynamic viscoelasticity measurement at room temperature when a vibration having a frequency of 1 Hz is applied.

In another case, when the second acoustic transmission medium is a solid with flowability, a solid for which a tanδ obtained by dynamic viscoelasticity measurement at room temperature when a vibration having a frequency of 0.1 Hz is applied is 1.0 or more is preferred.

Examples of solids satisfying these conditions include flexible gels. The flexible gel is the same as the gel exemplifying the first acoustic transmission medium, and examples thereof include: a hydrogel obtained by combining a hydrophilic polymer material such as polyvinyl alcohol or polyacrylic acid with a cross-linking component such as borax, a layered clay mineral (clay), or a hyperbranched polymer material such as a dendritic polymer; and a polymer gel obtained by combining a polymer material such as acrylic resin or urethane resin with a polyfunctional compound serving as a corresponding cross-linking agent.

It is preferred to use a value of tanδ obtained at a temperature and a time scale at the time when the vibrating body and the culture substrate are mechanically aligned through use of the acoustic transmission medium. That is, in regard to measurement of tanδ, it is possible to calculate tanδ through calculation using a relational expression of tanδ=G"/G' by measuring dynamic viscoelasticities G' and G" obtained when a vibration having a frequency of 1 (Hz) or 0.1 (Hz) is applied at room temperature, more preferably 25°C, by a rheometer or the like, where G represents a shear modulus, G' represents a storage shear modulus, and G" represents a loss shear modulus. However, it is sufficient that the above-mentioned conditions are satisfied when tanδ is measured for the second acoustic transmission medium, and tanδ may not be measurable for the second acoustic transmission medium in some cases. The second acoustic transmission medium may be a liquid at room temperature. In this case, tanδ may not be measurable.

When the second acoustic transmission medium is a liquid, the viscosity may be high to an extent that acoustic transmission is not impaired.

The viscosity being low is preferred in terms of ease of handling because an excess amount of liquid is easily discharged from between the culture substrate or the vibrating body and the acoustic transmission medium.

The viscosity can be measured at room temperature, more preferably 25°C, by a B-type viscometer or a rheometer. Further, it is preferred to adopt a value of the viscosity obtained at a shear rate of 1.0 (1/s) when measurement is performed at a gap of 1.0 mm through use of a 25-mm parallel plate. Depending on the viscosity of the liquid, an appropriate torque value may not be obtained under the above-mentioned conditions, but at that time, the measurement can be performed by changing values such as a diameter and a gap of the plate so as to achieve a similar shear rate.

Examples of the liquid include low-viscosity liquids such as water, physiological saline, ethanol, and diluted ethanol, and mixtures thereof, as well as highly viscous liquids such as glycerin, silicone oil, and polymer solutions, gel materials, and further mixtures thereof. A polymer solution in which a gel is dispersed or swollen in a liquid such as water may also be used. More specifically, an ultrasound gel in which additives such as a thickener, for example, a PVA gel, are added to a liquid such as glycerin to adjust viscoelasticity is also preferred. Gel materials may be classified as solids or liquids. A gel serving as a solid has a high content ratio of cross-linking components, and a gel serving as a liquid has a low content ratio of cross-linking components. Herein, materials for which tanδ has been measurable by the measurement method performed by the inventors are classified as solids, and materials for which tanδ has not been measurable are classified as liquids. This is considered to be related to a repulsive property of an elastic body. In the Examples below, PVA is a flexible gel serving as a solid, and an ultrasound gel is a gel serving as a liquid.

The second acoustic transmission medium is particularly preferably a hydrophilic liquid. For example, an ethanol/water mixture obtained by mixing water and ethanol in a volume ratio of from 3:7 to 2:8 is preferred because of having a high sterilizing activity and preventing bacterial growth. The second acoustic transmission medium preferably includes at least one type selected from the group consisting of water, physiological saline, ethanol, and diluted ethanol.

The second acoustic transmission medium may contain various additives for the purpose of controlling physical property values such as viscosity, affinity and acoustic impedance with the first acoustic transmission medium, surface tension, storage stability, and antibacterial activity.

Examples of the above-mentioned additives can include the following.

Examples of additives for adjusting surface tension can include anionic surfactants such as sodium dodecyl sulfate and sodium lauryl sulfate.

Examples of cationic surfactants can include ethyltrimethylammonium bromide, benzalkonium chloride, and dimethylaminopropylamide stearate.

Examples of nonionic surfactants can include Contaminon, Contaminon US, Pluronic (trademark) F-68, Tween 20, and Tween 80.

Examples of amphoteric surfactants can include CHAPS and CHAPSO.

Examples of storage stabilizers can include light stabilizers such as a hindered amine, benzophenone, and benzotriazole, and antioxidants such as amine-based, phenol-based, sulfur-based, and phosphorus-based antioxidants.

Examples of additives exhibiting antibacterial activity include benzalkonium chloride, sodium azide, and antibiotics such as ampicillin, penicillin, and streptomycin.

(Combination of Acoustic Transmission Media)

The second acoustic transmission medium fills unaligned portions when the first acoustic transmission medium and the culture substrate, or the first acoustic transmission medium and the vibrating body, are mechanically aligned. For that reason, the first acoustic transmission medium and the second acoustic transmission medium complement each other so that an air layer is less likely to be present between the acoustic transmission medium and the culture substrate or the vibrating body. Preferred examples of a combination of the first acoustic transmission medium and the second acoustic transmission medium can include an example in which the first acoustic transmission medium includes silicone rubber and the second acoustic transmission medium includes at least one type selected from the group consisting of water, physiological saline, ethanol, and diluted ethanol.

It is preferred to vibrate the vibrating body under a state in which, on a surface of the culture substrate or the vibrating body that is in contact with the first acoustic transmission medium and the second acoustic transmission medium, a ratio of the area of a portion in contact with the first acoustic transmission medium to the total area of portions in contact with the first and second acoustic transmission media is 5% or more.

That is, it is preferred to satisfy at least one of:

$$0.05 \leq S1/S1+S2,$$

where S1 is the area of the portion in which the culture substrate is in contact with the first acoustic transmission medium, and S2 is the area of the portion in which the culture substrate is in contact with the second acoustic transmission medium; or

$$0.05 \leq S3/S3+S4,$$

where S3 is the area of the portion in which the vibrating body is in contact with the first acoustic transmission medium, and S4 is the area of the portion in which the vibrating body is in contact with the second acoustic transmission medium.

When the area of the portion in which the first acoustic transmission medium is mechanically aligned (the area of the portion in which the vibrating body is in contact with the first acoustic transmission medium) is 5% or more, the vibrating body or the culture substrate can be brought into close contact with the first acoustic transmission medium that is a solid, and the vibration transmission path is kept constant, thereby further facilitating vibration control so as to easily achieve desired vibration. S1/S1+S2 and S3/S3+S4 may be referred to as "contact area ratio" or "contact area ratio of the first acoustic transmission medium." Those contact area ratios may also be expressed as a ratio or expressed as a percentage. That is, $0.05 \leq S1/S1+S2$ or $0.05 \leq S3/S3+S4$ may be expressed as, for example, the contact area ratio being 0.05 or more, or the contact area ratio of the first acoustic transmission medium being 5% or more.

When the area of the portion in which the first acoustic transmission medium is mechanically aligned (the area of the portion in which the vibrating body is in contact with the first acoustic transmission medium) is less than 5%, the area for bringing the vibrating body and the culture substrate into close contact with each other through intermediation of a solid is reduced, and it may consequently become difficult to perform control so as to achieve desired vibration.

As a method of calculating the area of the portion in which the first acoustic transmission medium and the second acoustic transmission medium are in contact with each other, for example, there is a method of calculating the area by coloring the second acoustic transmission medium and thereby determining the first acoustic transmission medium to be in an uncolored region and the second acoustic transmission medium to be in a colored region to calculate the area. There is also a method of creating a contrast difference in refractive index difference at a portion in which the first and second acoustic transmission media are in contact with each other by adjusting, for example, illumination during observation, and calculating the area of a region exhibiting a different contrast. There is also, for example, a method of measuring a lateral frictional force after the vibrating body and the culture substrate are brought into close contact with each other through intermediation of a solid, and thereby estimating the contact area of the first acoustic transmission medium based on an increase amount of the frictional force.

The portion in which the first acoustic transmission medium and the culture substrate are mechanically aligned is a region that can well transmit the vibration of the vibrating body to the culture substrate. In order to efficiently transmit the vibration of the vibrating body to the culture substrate, it is preferred that an antinode (region exhibiting a large amplitude value) in a vibration pattern of the vibrating body overlap with the region in which the first acoustic transmission medium and the culture substrate are mechanically aligned. When the antinode in the vibration pattern of the vibrating body does not overlap with the region in which the first acoustic transmission medium and the culture substrate are mechanically aligned, vibration transmission is hindered, and cell detachment efficiency may decrease or variation at the time of detachment may increase.

In order to mechanically align a desired region, for example, there is used a method in which the desired region on the culture substrate surface of the first acoustic transmission medium is designed to be convex compared to the surrounding area. Specifically, for example, to efficiently transmit a pattern having strong vibration in the center, a central portion of the first acoustic transmission medium is formed to be convex in shape, and to efficiently transmit a pattern having strong vibration in a peripheral portion, the peripheral portion of the first acoustic transmission medium is formed to be convex in shape. Examples of processing methods include cutting and processing a flat silicone rubber or the like, and preparing a mold for any surface shape in advance and performing molding processing.

The volume ratio of the second acoustic transmission medium to the first acoustic transmission medium is preferably 0.05 or more and 2.0 or less. When the volume ratio is less than 0.05, a volume of the second acoustic transmission medium is small, and when the first acoustic transmission medium and the culture substrate are mechanically aligned, the amount of the first acoustic transmission medium for filling the unaligned portions may be insufficient, thereby potentially hindering vibration transmission.

When the volume ratio is greater than 2.0, the volume of the second acoustic transmission medium increases, and an excessive portion of the second acoustic transmission medium may overflow to the surroundings during the above-mentioned alignment. If the overflowed second acoustic transmission medium directly contacts both the vibrating body and the culture substrate, this may result in undesirable effects such as an increase in the number of vibration transmission paths or suppression of vibration due to contact with the culture substrate, thereby making it difficult for the vibration of the vibrating body to be properly transmitted.

In the arrangement step, even when the second acoustic transmission medium is excessively arranged, it suffices that the excessive portion of the second acoustic transmission medium can be removed thereafter. Examples of methods for removing the excessive portion of the second acoustic transmission medium include the following methods.

Through use of a vibrating plate 11 having a protruding shape as illustrated in Fig. 6B as the vibrating body, the excessive portion of the second acoustic transmission medium is more easily discharged without staying around the acoustic transmission medium.

As illustrated in Fig. 11, a first acoustic transmission medium 131 can be structured to include, in a surface thereof facing the culture substrate or the vibrating body, a continuous groove portion 30 extending from a central portion to an outer peripheral portion of the surface, thereby facilitating discharge of the excessive portion of the second acoustic transmission medium to the outside through each groove.

In another case, a contact angle between the first and second acoustic transmission media can be preferably 120° or less, more preferably 90° or less.

When the contact angle between the first and second acoustic transmission media becomes smaller, the excessive portion of the second acoustic transmission medium becomes less likely to stay near the aligned portion, and hence a vibration transmission path due to the excessive portion of the second acoustic transmission medium becomes less likely to be caused.

In addition, a contact angle between the second acoustic transmission medium and a surface of the culture substrate or the vibrating body in a region in which the culture substrate or the vibrating body is in contact with the second acoustic transmission medium can be set to be less than 40°.

In another case, in order for the excessive portion of the second acoustic transmission medium to be less likely to stay near the aligned portion, it is possible to perform surface treatment on any one of a surface of the culture substrate facing the first acoustic transmission medium, a surface of the vibrating body facing the first acoustic transmission medium, a surface of the first acoustic transmission medium facing the culture substrate, or a surface of the first acoustic transmission medium facing the vibrating body, and this step can be called a surface treatment step. The excessive portion of the second acoustic transmission medium becomes less likely to stay near the aligned portion, and hence a vibration transmission path due to the excessive portion of the second acoustic transmission medium becomes less likely to be caused.

Examples of the surface treatment can include hydrophilization treatment, and more specific examples thereof include laser treatment, chemical treatment, and plasma treatment.

The vibration path that is caused by the excessive portion of the second acoustic transmission medium may also be caused when the vibrating body and the culture substrate are directly aligned by the second acoustic transmission medium without intermediation of the first acoustic transmission medium. Thus, when the contact angle between the second acoustic transmission medium and the surface of the culture substrate or the vibrating body in the region in which the culture substrate or the vibrating body is in contact with the second acoustic transmission medium becomes smaller, the excessive portion of the second acoustic transmission medium on the surface that is in contact with the second acoustic transmission medium is more easily discharged.

The method for removing the excessive portion of the second acoustic transmission medium as listed above can also be a combination of a plurality of methods, and in this case, a further increased effect may be exhibited.

Further, examples of method for adjusting the amount of the second acoustic transmission medium during alignment include forming the first acoustic transmission medium of a porous resin material. Through impregnation of the second acoustic transmission medium into the porous resin material, it is possible to mechanically align the acoustic transmission medium and the culture substrate by allowing the second acoustic transmission medium to ooze out to the aligned surface.

The degree of oozing of the second acoustic transmission medium during alignment is controlled by a pore diameter and a pore diameter distribution in a height direction of voids contained in the porous resin material, a proportion of the voids, and also an amount of pressure applied in the pressing step.

Further, an amount of volatilization of the unused second acoustic transmission medium obtained when the detachment step has not been performed can be controlled by the pore diameter and the pore diameter distribution in the height direction of the voids contained in the porous resin material, and the proportion of the voids, as well as the pore diameter of voids on a surface of the resin material and a proportion of the voids.

As a method of manufacturing the porous resin material, a publicly-known manufacturing method is used, and examples thereof include a method of manufacturing a porous resin material by adding a salt having a uniform particle size to a resin raw material, and a method of manufacturing a porous resin material by dispersing and stabilizing fine liquid droplets such as a solvent in the resin raw material.

In regard to the contact surface between the culture substrate and the first acoustic transmission medium or the contact surface between the vibrating body and the first acoustic transmission medium, in order to improve the alignment, the entire surface or a partial surface of any one of the contact surface between the culture substrate and the first acoustic transmission medium or the contact surface between the vibrating body and the first acoustic transmission medium may be bonded through use of an adhesive or a pressure-sensitive adhesive, for example. When the above-mentioned (a) is satisfied and the (b) is not satisfied, a configuration in which the vibrating body and the first culture substrate are bonded to each other with an adhesive or the like can be adopted, and when the above-mentioned (b) is satisfied and the (a) is not satisfied, a configuration in which the vibrating body and the first culture substrate are bonded to each other with an adhesive or the like can be adopted. However, the culture substrate is often used disposable, and hence the configuration in which the vibrating body and the first acoustic transmission medium are bonded to each other with an adhesive or the like is preferred to the configuration in which the culture substrate and the first acoustic transmission medium are bonded to each other with an adhesive or the like.

Accordingly, in a case of bonding any one of the culture substrate or the vibrating body to the first acoustic transmission medium, it is preferred to bond the vibrating body to the first acoustic transmission medium. As the adhesive or pressure-sensitive adhesive, a publicly-known adhesive, pressure-sensitive adhesive, double-sided tape, or the like is used.

(Culture Substrate)

In the cell detachment method according to the present disclosure, cells adhering to a culture surface of a culture substrate are detached from the culture substrate. The term "cell adherence" includes not only adhesion of adherent cells but also cells simply in contact with the surface. That is, the phrase "cell adherence to the culture substrate" includes adhesion of cells thereto through adhesion factors, but is not limited thereto, and includes cells remaining on the surface due to weak interaction with the culture substrate, or cells simply remaining due to external forces such as gravity.

The term "culture substrate" as used in the present disclosure refers to any substrate for use in cell culturing that includes a surface to which cells adhere. The culture substrate includes, for example, a container for cell culturing or a part thereof. Examples of a cell container can include a dish (also called a Petri dish), a flask, a plate, and a tube. For all such purposes, products from companies such as, for example, Corning Incorporated, Thermo Fisher Scientific Inc., and AGC Techno Glass Co., Ltd. are used as general-purpose disposable containers. Of those, a dish is typical as an example of the culture substrate in the present disclosure. Culture substrates provided with a removable lid are commonly used. The culture substrate as used herein may or may not include parts such as a lid. An example of a shape of a dish is as illustrated in the schematic view of Fig. 8. In this illustrated configuration, in particular, a bottom surface warp height 300 varies greatly among model numbers of different manufacturers, among production lots, and even within the same production lot. It is required to take variation in the bottom surface warp height 300 into consideration in order to mechanically align the culture substrate with the acoustic transmission medium, but mechanical alignment is facilitated through use of the first acoustic transmission medium and the second acoustic transmission medium in the present disclosure in combination.

As illustrated in Fig. 8, a bottom surface of a commercially available culture substrate has a projecting portion on an outer peripheral portion thereof. Main roles of the projecting portion are considered to be, for example, preventing damage to the bottom surface of the substrate, enabling containers to be stacked, and preventing a temperature of a work bench from being directly transferred to the bottom surface of the substrate at placement on the work bench. When the culture substrate and the acoustic transmission medium are mechanically aligned, the alignment may be performed by avoiding this projecting portion or by covering the projecting portion.

A material for the culture substrate is only required to be a material that is chemically stable and capable of culturing desired cells, and examples thereof include polyethylene, polypropylene, polycarbonate, polystyrene, polyvinyl chloride, nylon, polyurethane, polyurea, polylactic acid, polyglycolic acid, polyvinyl alcohol, polyvinyl acetate, poly(meth)acrylic acid, poly(meth)acrylic acid derivatives, polyacrylonitrile, poly(meth)acrylamide, poly(meth)acrylamide derivatives, polysulfone, cellulose, cellulose derivatives, polysilicone, polymethylpentene, glass, and metals. Of those, polystyrene is preferred.

(Vibrating Body)

The vibrating body in the present disclosure is used without limitation as long as the vibrating body generates vibration, and examples thereof include a vibrating body obtained by bonding a piezoelectric body and a vibrating plate to each other. When the piezoelectric body is circular, it is preferred to use glass, stainless steel (SUS), or quartz for the vibrating plate. Through use of glass, stainless steel (SUS), or quartz for the vibrating plate, the vibrating body can output a large amplitude at a relatively high driving frequency (vibration frequency) in the ultrasonic region without damage. The vibrating body can include an ultrasonic vibrator.

In a case of a ring-shaped piezoelectric body, it is preferred that an outer diameter dimension of the vibrating plate be equal to an outer diameter dimension of the piezoelectric body.

In regard to a thickness of the vibrating plate, it is preferred that a midpoint in a thickness direction of flexure, namely, a neutral plane which is neither in tension nor in compression during flexure, be on the vibrating plate side when the piezoelectric body and the vibrating plate are bonded to each other and flexurally vibrate, in order to efficiently use strain of the piezoelectric body for the flexure.

Examples of a shape of the vibrating plate in the present disclosure include a flat-plate-shaped vibrating plate 11 as illustrated in Fig. 6A and a protruding-shaped vibrating plate 11 as illustrated in Fig. 6B. Examples of a method of manufacturing a protruding-shaped vibrating plate include a method of bonding vibrating plates different in diameter and a method of performing processing so as to form a protruding shape through cutting processing.

(Piezoelectric Body)

The piezoelectric body that is used in the vibrating body in the present disclosure is preferably a ring-shaped piezoelectric body, but the shape of the piezoelectric body that causes the vibrating body to undergo flexure may be an annular shape or a disc shape. In order to directly observe the cell detachment state, the annular shape that enables a field of view below the culture substrate to be secured is preferred.

The piezoelectric body exhibits piezoelectric characteristics by being subjected to poling treatment, but, as illustrated in Fig. 10, the polarity of the poling may be changed depending on an electrode pattern. Portions in which electrodes A1, A2, A3, B1, B2, and B3 are patterned are driving phases that contribute to deformation, and a region sandwiched between the electrodes A1 and B1 is a sensor phase for detecting a degree of deformation. The piezoelectric body subjected to the poling treatment can excite vibration modes of a standing wave mode and a traveling wave mode by controlling a phase of an input AC voltage for respective electrode patterns. A standing wave is generated by applying an AC voltage with a 180-degree phase difference to the negatively poled electrodes (A2 and B2) with respect to the positively poled electrodes (A1, A3, B1, and B3). In contrast, when the electrodes are divided into an A phase (electrodes A1, A2, and A3) and a B phase (electrodes B1, B2, and B3) on the left and right of the one-dot chain line as illustrated in Fig. 10, a two-phase-driven traveling wave is generated by applying an AC voltage with a 90-degree phase difference between the A phase and the B phase. In Fig. 10, GND represents ground, and is used for grounding the electrode arranged on the back surface.

An example of forming the electrode patterns through use of Ag by a printing method can be given, but an electrode material may not only be Ag but also a noble metal such as Au, Pt, or Pd, or a base metal such as Cu. A forming method therefor may be a printing method, a plating method, or a sputtering method.

$PbZrTiO_3$ (PZT) was used for a material composition of the piezoelectric body used in the vibrating body, but a lead-free piezoelectric material that substantially does not use Pb is preferred in consideration of environmental regulations. Examples of a main component of the lead-free piezoelectric material include $BaTiO_3$ (BT), $NaNbO_3$, $BiNaTiO_3$, and $BiFeO_3$, and a combination thereof or a metal element may be added to the main component. In particular, vibration performance equivalent to that obtained with $PbZrTiO_3$ (PZT) was confirmed with a $BaTiO_3$ (BT)-based material. In addition, as materials other than ceramics, single crystal materials or polymer-based piezoelectric materials may be used.

In regard to a thickness of the piezoelectric body of the vibrating body, it is preferred that the midpoint in the thickness direction of the flexure, namely, the neutral plane which is neither in tension nor in compression during the flexure, be on the vibrating plate side when the bonded and laminated piezoelectric body and vibrating plate flexurally vibrate, in order to efficiently use the strain of the piezoelectric element for the flexure, and hence a combination of the thicknesses of the piezoelectric body and the vibrating plate was calculated based on a relationship between the hardness of the piezoelectric body and the hardness of the vibrating plate.

(Steps)

The cell detachment method according to the present disclosure is characterized by including the following steps.

Arrangement step:

A step of arranging the first acoustic transmission medium and the second acoustic transmission medium between the culture substrate and the vibrating body. Detachment step:
A step of detaching the cells from the culture surface by vibrating the vibrating body under a state in which at least one of (a) or (b) is satisfied:

(a) the culture substrate is in contact with the first acoustic transmission medium and the second acoustic transmission medium; or
(b) the vibrating body is in contact with the first acoustic transmission medium and the second acoustic transmission medium.

Further, a pressing step can be given as an additional step. The pressing step is a step of applying pressure so as to bring the culture substrate and the vibrating body closer to each other, and this step promotes contact between the culture substrate or the vibrating body and the first acoustic transmission medium and the second acoustic transmission medium.
One form of a specific example of the cell detachment method according to the present disclosure is illustrated in Fig. 5B.
First, as the arrangement step, the first acoustic transmission medium is placed on the vibrating body (Step S210).
After that, the second acoustic transmission medium is placed on the first acoustic transmission medium (Step S220).
Next, as the pressing step, the first and second acoustic transmission media are brought into contact with the culture substrate (Step S230). After that, pressure is applied from above the culture substrate through use of a weight or the like (Step S240).
As the detachment step, ultrasonic application conditions are set (Step S250). Next, ultrasonic application is started (Step S260). Finally, the detached cells are collected (Step S270).
The application of pressure is not an essential step for the cell detachment method according to the present disclosure.
Each step is described below.

(Arrangement Step)

In the arrangement step, the first acoustic transmission medium and the second acoustic transmission medium are each arranged so as to be in contact with the vibrating body or the culture substrate in accordance with a state of vibration generated by the vibrating body in the detachment step. Further, in the detachment step, any one of the first acoustic transmission medium or the second acoustic transmission medium can be arranged so as to be in contact with any one of the vibrating body or the culture substrate based on the state of vibration to be excited in the vibrating body in the detachment step.
For example, among states of vibration to be excited in the vibrating body, when it is desired to cause the central portion of the culture substrate to conform to the state of vibration of the vibrating body, it is preferred to use the first acoustic transmission medium having such a shape that the central portion of the culture substrate comes into contact therewith.
The second acoustic transmission medium is applied in advance to the portion in which the culture substrate and the first acoustic transmission medium are not in contact with each other, thereby enabling contact with the first and second acoustic transmission media.
As an application method, it is preferred to adopt a method that can apply a volume that is neither excessive nor insufficient relative to the area of the contacting portion and a resulting gap, in a manner that ensures small variation. Examples thereof include a method of spraying and applying the second acoustic transmission medium through use of a dispenser, a nozzle, a spray, or the like, and a method of impregnating a sponge or the like with the second acoustic transmission medium and applying the second acoustic transmission medium.

(Pressing Step)

The pressing step in this embodiment may be any step of applying pressure so as to bring the culture substrate and the vibrating body closer to each other. The culture substrate may be displaced to approach the vibrating body, or the vibrating body may be displaced to approach the culture substrate, or both the culture substrate and the vibrating body may be displaced to be brought closer to each other.

In the pressing step, pressure is applied from the culture substrate side or the vibrating body side so as to mechanically align the culture substrate and the acoustic transmission medium by bringing the culture substrate or the vibrating body into contact with the first acoustic transmission medium and the second acoustic transmission medium.

Examples of pressing unit for performing the pressing step include a method of placing a weight on the lid of the culture substrate, and a method of applying a load upward from below the vibrating body while an upper portion of the culture substrate lid is fixed from above so as not to move by applying a uniform load from above by a loading method such as a spring or a leaf spring from above the lid of the culture substrate container.

For the pressing unit, a load by a weight is preferred in consideration of vibration insulation, but other loading methods can be selected to an extent that does not inhibit vibration. For example, a constant-force spring can be used as the pressing unit.

In regard to a shape of the weight to be placed on the lid of the culture substrate, it is preferred to use a weight having, for example, an annular shape so that cells in the container can be easily observed.

The load to be applied varies depending on the size of the culture substrate and the vibration pattern to be applied. For example, in a case of a 35-mm dish, the load to be applied thereto is preferably from 10 g to 300 g, and in a case of a 60-mm dish, the load to be applied thereto is preferably from 100 g to 600 g. The load may be applied through use of a weight, or may be the weight of the culture container itself.

When the weight is excessively heavy, in addition to suppressing and inhibiting the vibration itself, the culture substrate itself may undergo flexure, resulting in a non-uniform vibration transmission state, thereby causing a decrease in the detachment rate.

(Detachment Step)

In the detachment step, cells are detached by vibrating the vibrating body under a state in which the culture substrate or the vibrating body is in contact with the first acoustic transmission medium and the second acoustic transmission medium. Further, the detachment step can include a step of exciting vibration in an ultrasonic band in the vibrating body.

In the arrangement step, the vibration of the vibrating body is not started, and the vibration of the vibrating body is started in the detachment step. For example, in the examples of the device illustrated in Fig. 6A and Fig. 6B, the vibrating body is driven by supplying a voltage at a resonance frequency to generate a desired ultrasonic vibration mode. In this case, in the detachment step, the vibrating body is driven by supplying a voltage at a resonance frequency to the vibrating body to generate a desired ultrasonic vibration mode. A power supply that supplies the above-mentioned voltage is not shown in Fig. 6A and Fig. 6B.

The vibration pattern can be freely selected depending on a cell type and a detachment result. Further, such a vibration pattern may be used singly, or may be input by being periodically changed, in the detachment step.

It is preferred that the vibration waveform can cause the cell culture surface of the culture substrate to vibrate at a constant amplitude, for example, an amplitude of 1 μm. Basically, resonance vibration can be detected when a frequency sweep is performed from a low frequency to a high frequency, for example, from 20 kHz to 150 kHz in the ultrasonic region, but the vibration detected on the low-frequency side has a large amplitude but a small acceleration. In contrast, the vibration detected on the high-frequency side has a small amplitude but a large acceleration. From the above, conditions for applying a force to weaken an adhesion force of the cultured cells to the culture substrate depend greatly on the adhesion force of the cells, and a size and a shape of the cells. In addition, the flow generated in a liquid such as a detachment liquid in the cell culture substrate also differs depending on the amplitude and the acceleration, and hence various combinations exist. Further, simply a large amplitude and a large acceleration may not be preferred because of promoting occurrence of cavitation serving as a factor in reducing cell viability.

The vibration waveform can be set to a standing wave mode formed as such a pattern that has an order concentrically from the center of the disc-shaped vibrating plate or in a circumferential direction thereof, or to a traveling wave mode that forms a radial vibration waveform on the vibrating plate by vibrating the piezoelectric body with the phase changed in the radial direction. For each mode, the vibration waveform can be formed by applying a voltage at a resonance frequency to the vibrating body, and the resonance frequency and a shape of the vibration waveform can be calculated by structural calculation using the shape of the vibrating body and material physical property values thereof such as hardness and density.

Further, when the vibrating body is continuously driven, the resonance frequency shifts to the low-frequency side due to heat generation of the piezoelectric body itself, and hence, as the driving is continued at a fixed frequency, the amplitude becomes smaller. For that reason, a sufficient amplitude can be obtained through the driving using a driving method that suppresses a temperature rise due to self-heating by providing a rest time after the vibration is performed for a certain period of time, or a driving method that repeatedly sweeps from the high-frequency side to the low-frequency side with a fixed frequency width such that the resonance frequency is always passed even when heat is generated.

(Vibration Evaluation)

The vibration waveform of the vibrating plate can be measured with a laser Doppler vibrometer (AT7200 from Graphtec Corporation). A measurement method is described by taking the cell detachment device illustrated in Fig. 6A as an example. When the cell detachment device is driven at a desired resonance frequency and voltage and the surface of the vibrating plate 11 is two-dimensionally scanned through use of a laser in the X and Y directions, a vibration velocity at each point can be obtained, and an amplitude in the Z direction can be obtained by calculation. As a result, the vibration waveform of the vibrating plate 11 can be three-dimensionally constructed, and positions of antinodes exhibiting large vibration amplitudes and nodes exhibiting no vibration during driving can be dynamically grasped.

The vibration of the cell culture surface of a culture substrate 21 can also be measured with a similar configuration. It is preferred to place the acoustic transmission medium 13 on the vibrating plate 11, form a film of a metal, for example, Ag, on an inner surface of the culture substrate 21 by vapor deposition so that a laser light is reflected by the culture substrate 21, and to place the culture substrate 21 such that an air layer is not introduced between the culture substrate 21 and the acoustic transmission medium 13. In Fig. 6A, the first acoustic transmission medium and the second acoustic transmission medium are collectively illustrated as the acoustic transmission medium 13.

In a case of a standing wave mode in which a target resonance to be obtained through driving is a resonance that exhibits a concentric amplitude pattern, the amplitude of the central portion of the culture substrate 21 placed on the acoustic transmission medium 13 is swept around the target resonance frequency by the cell detachment device, while the entire cell culture surface of the culture substrate 21 is two-dimensionally scanned at a frequency at which the amplitude is maximum, and hence the vibration waveform generated on the cell culture surface of the culture substrate 21 can be three-dimensionally grasped.

(Cells)

The cell detachment method according to the present disclosure can be applied to all types of cells without limitation. Examples thereof include various cultured cell lines such as Chinese hamster ovary-derived CHO cells, mouse connective tissue L929 cells, mouse skeletal muscle myoblasts (C2C12 cells), human fetal lung-derived normal diploid fibroblasts (TIG-3 cells), human fetal kidney-derived cells (HEK293 cells), human alveolar basal epithelial adenocarcinoma-derived A549 cells, human cervical cancer-derived HeLa cells, and insect-derived cell lines, epithelial cells and endothelial cells forming various tissues and organs in vivo, skeletal muscle cells, smooth muscle cells, and cardiac muscle cells that exhibit contractility, neuron cells and glial cells forming the nervous system, fibroblasts, primary cultured cells such as hepatic parenchymal cells, non-parenchymal hepatic cells, and adipocytes involved in metabolism in vivo, various stem cells such as induced pluripotent stem (iPS) cells, embryonic stem (ES) cells, embryonic germ (EG) cells, embryonal carcinoma (EC) cells, mesenchymal stem cells, hepatic stem cells, pancreatic stem cells, skin stem cells, muscle stem cells, and germ stem cells, and precursor cells of each tissue, and further, cells induced to differentiate therefrom.

Cell culture conditions can be selected appropriately in accordance with the cells to be cultured. In general, an appropriate medium is added into the culture substrate 21, and cells of from about $1.0\times10^1$ cells/cm$^2$ to about $5.0\times10^4$ cells/cm$^2$ are seeded therein and cultured in an environment of 37°C and a $CO_2$ concentration of 5%. At this time, it is preferred to culture until a cell occupancy ratio in the culture substrate 21 reaches about 70% to about 80%, which is a so-called subconfluent state.

A culture medium (medium) of the cultured cells can be replaced by a cell detachment liquid in the cell detachment step.

In general, the detachment liquid contains a proteolytic enzyme. Examples of proteolytic enzymes include trypsin, Accutase, collagenase, natural protease, chymotrypsin, elastase, papain, pronase, and recombinants thereof. However, proteolytic enzymes may dissolve cells, and hence there is concern about adverse effects on cells. In contrast, the cell detachment method according to the present disclosure exhibits a similar effect without use of a proteolytic enzyme or with only an extremely small amount of a proteolytic enzyme even when used, and thus has an effect of suppressing adverse effects on cells.

The cell detachment liquid is a liquid to be used at the time of detachment of cells, and may have an action of reducing the adhesion force of the cells.

A pH of the cell detachment liquid is preferably in a neutral range. This is because the neutral range is suitable for cell culturing and can keep the cell viability stably high. The pH can be adjusted appropriately with hydrochloric acid, sodium hydroxide, or the like. Further, various buffer solutions are suitably used to stably maintain the pH. The detachment liquid may also be a medium.

The viscosity of the cell detachment liquid can be adjusted appropriately by, for example, adding polymers or sugars.

A solution containing a metal ion chelating agent (which may hereinafter be referred to as "chelating agent") is particularly preferred as the cell detachment liquid. This is because the adhesion force of cells can be effectively reduced through use of a cell detachment liquid containing a chelating agent.

The chelating agent is not particularly limited, but examples thereof include ethylenediaminetetraacetic acid (which may hereinafter be referred to as "EDTA"), ethylenediamine, ethylenediaminetetramethylenephosphonic acid, glycol ether diaminetetraacetic acid, nitrilotriacetic acid, diethylenetriaminepentaacetic acid, iminodiacetic acid, dihydroxyethylglycine, dicarboxymethylglutamic acid, ethylenediaminedisuccinic acid, etidronic acid, citric acid, gluconic acid, and phosphonobutane triacetic acid. Of those, a chelating agent that forms a chelate with a divalent cation is preferred, a chelating agent that forms a chelate with $Ca^{2+}$ and $Mg^{2+}$ is particularly preferred, and ethylenediaminetetraacetic acid is most preferred. When ethylenediaminetetraacetic acid is used as the chelating agent, the pH of the cell detachment liquid is preferably 7.0 or more and 8.0 or less. This is because a chelation ability of ethylenediaminetetraacetic acid can be enhanced by a slightly higher pH in the neutral range that allows the cell viability to be kept high, and thus efficiency of reducing cell adhesion force can be further increased. The chelating agent may be used alone or in combination of two or more types.

The content of the chelating agent is preferably 0.01 mM or more and 5.0 mM or less.

When the content falls within this range, a chelation effect can be reliably obtained, and a decrease in activity due to presence of an excessive amount of the chelating agent can also be suppressed.

The cell detachment liquid may contain a hydrophilic polymer including a polyalkylene glycol structure. An example of the hydrophilic polymer including a polyalkylene glycol structure is polyethylene glycol. The hydrophilic polymer preferably has a peak molecular weight Mp measured by gel permeation chromatography of 800 or more and 50,000 or less, more preferably 1,200 or more and 20,000 or less. This is because an influence of the polymer on cells is small, and a viscosity-increasing effect due to the polymer can be suppressed.

(Detachment Test)

A detachment test can be performed in order to evaluate the cell detachment method according to the present disclosure. The detachment test can be performed, for example, as follows.

The arrangement step can be performed on a substrate on which cells are cultured and, when required, the culture medium (medium) is replaced by the detachment liquid. When required, the vibrating body can also be driven while the substrate is kept at a constant temperature, for example, 25°C, in an incubator. The driving is performed for a time sufficient for cell detachment. After the driving, the detached cells are collected with a pipette, and cells failing to be collected and still adhering to the culture substrate 21 are collected by pipetting. Cells may be damaged by being left in a high-temperature environment after removal of the culture medium (medium). Further, some cells are damaged by being exposed to changes in environmental temperature, and hence it is preferred to set a temperature at the time of placing the cells in the detachment device or at the time of detachment of the cells in accordance with characteristics of the cells to be used.

In regard to various measurement values described later, the physical properties of the acoustic transmission medium at the temperature at the time of placing the cells provided in the culture container in the detachment device are particularly important. Thus, the first acoustic transmission medium is a solid at room temperature, and the second acoustic transmission medium is a liquid or a solid with flowability at room temperature. Further, it is preferred that the first acoustic transmission medium be a solid and the second acoustic transmission medium be a liquid or a solid with flowability even at the temperature at the time of detachment. It is also preferred that the second acoustic transmission medium be a solid having a tan$\delta$ of 1.0 or more at room temperature when a vibration having a frequency of 0.1 Hz is applied.

The detachment rate used below is expressed as a percentage of the number of cells detached by vibration with respect to the total number of cells detached by vibration and cells detached by pipetting. For the variation, the detachment rate was determined at n=10, and a standard deviation of the detachment rate was evaluated as A for less than 5%, B for 5% or more and less than 7.5%, C for 7.5% or more and less than 10%, and D for 10% or more.

Residual cells remaining on the bottom surface of the substrate after cell detachment can be observed through use of scattered light from a backlight.

(Cell Detachment Device)

The cell detachment device according to the present disclosure is a cell detachment device for detaching cells from a culture surface of a culture substrate by transmitting vibration generated by a vibrating body to the cells provided on the culture surface, the cell detachment device including:

a vibrating body;
a first acoustic transmission medium; and
a culture substrate placement portion, in the stated order, and
the cell detachment device further including supply unit for supplying a second acoustic transmission medium,
in which the supply unit supplies the second acoustic transmission medium so that at least one of the following (a) or (b) is satisfied:

(a) the culture substrate is in contact with the first acoustic transmission medium and the second acoustic transmission medium; or
(b) the vibrating body is in contact with the first acoustic transmission medium and the second acoustic transmission medium.

The configuration of the cell detachment device according to the present disclosure is illustrated in Fig. 6A.

As illustrated in Fig. 6A, the cell detachment device according to the present disclosure includes a vibrating body 101, the acoustic transmission medium 13, and supply unit 23 for supplying the second acoustic transmission medium. In this figure, the first acoustic transmission medium 131 and the second acoustic transmission medium supplied by the supply unit 23 are illustrated collectively as the acoustic transmission medium 13. The cell detachment device can further include an arm 24 for moving the supply unit 23 in the X, Y, and Z axis directions. The culture substrate placement portion is only required to be recognized by a user as a place to put the culture container. In the example of Fig. 6A, an opening defined by the vibrating body 101, an upper cover 15, and a cushioning material 16 is suitable for placing a general-purpose dish, and hence this opening corresponds to the culture substrate placement portion.

In the cell detachment device of Fig. 6A, an example in which the vibrating body 101 serving as the vibration generation portion includes a piezoelectric body 12 and the vibrating plate 11 is illustrated. The vibrating body 101 including the piezoelectric body 12 and the vibrating plate 11 generates flexural vibration in which the vibration is amplified by a combination of hardness of the piezoelectric body 12 that expands and contracts itself and hardness of the vibrating plate 11 bonded thereto. In this example, resonance due to structural factors of the vibrating body 101 is used, and large vibration can be obtained at a resonance frequency. The vibrating body can include a vibrator such as an ultrasonic vibrator. The above-mentioned expansion and contraction are performed by applying a voltage to the piezoelectric body 12 through use of a power supply (not shown) (the same holds true for Fig. 6B and Fig. 6C).

In Fig. 6A, an annular-shaped vibrator is illustrated as a representative of the vibrating body 101, but a Langevin vibrator or a rectangular vibrator which can generate a large amplitude may also be used.

In order to efficiently transmit the vibration formed on the vibrating plate by resonance to the culture substrate 21 on which cells are cultured, the acoustic transmission medium 13 is interposed between the vibrating plate 11 and the culture substrate 21. When the culture substrate 21 is placed directly on the vibrating plate 11, a contact portion is in a state of point contact, and energy is dissipated as non-vibrational energy caused by, for example, squealing or heat generation, thereby causing a decrease in efficiency. The acoustic transmission medium 13 is basically preferably placed over an area sufficient to cover an area for detaching the cells adhering to the culture substrate 21.

In order to fix the vibrating body 101, a structure in which an end portion of the vibrating body 101 is sandwiched by the upper cover 15, a lower cover 17, the cushioning material 16, and bolts 18 of Fig. 6A is adopted, but pressure for sandwiching the end portion is set to a pressure that allows tightening to an extent that does not inhibit the vibration generated in the vibrating plate.

An example in which a weight 14 serving as the pressing unit is provided above the culture substrate and below the vibrating body is illustrated. The application of pressure and release of the pressure are enabled by the weight 14 moving up and down. The pressing unit applies pressure so as to bring the culture substrate and the vibrating body closer to each other.

Fig. 6B is a view of another example. In Fig. 6B, the vibrating plate 11 has a protruding shape, and the excessive portion of the second acoustic transmission medium is more easily discharged without staying around the acoustic transmission medium.

A desired ultrasonic vibration mode is generated by driving the vibrating body by supplying a voltage at the resonance frequency.

For driving the vibrating body, the cell detachment device can be driven through use of an AC power supply that supplies a voltage at the resonance frequency, and through application of applying a voltage at a frequency of a vibration mode capable of forming a desired vibration waveform on the cell culture surface of the culture substrate, at which a desired amplitude is obtained. At this time, when a voltage of an S phase, which is the sensor phase provided to the vibrating body is measured, with an oscilloscope, the voltage generated through strain of the vibrating body itself can be directly monitored, and the driving can be performed while the state of vibration of the vibrating body is being examined. Further, when a thermocouple that can measure the temperature during the cell detachment step is installed at a location that does not affect the vibration, the driving can be performed while the temperature is being monitored. An infrared thermometer may also be used for temperature measurement.

The cell detachment device according to the present disclosure further includes an information processing device, and the information processing device can acquire information regarding the state of vibration to be excited in the vibrating body and control a supply unit based on the acquired information.

Fig. 6C is a conceptual view for illustrating the cell detachment device including the information processing device.

In Fig. 6C, an information processing device 205 acquires information regarding the state of vibration to be excited in the vibrating body 101. The information processing device 205 controls the arrangement and amount of the second acoustic transmission medium by controlling a supply unit 23 based on the acquired information so that cells are detached under optimal conditions.

The information processing device 205 includes a central processing unit (CPU) 206, a random access memory (RAM) 207, a read only memory (ROM) 208, and a hard disk drive (HDD) 209 in order to implement functions of a computer that performs calculation and storage. The information processing system 205 also includes a communication interface (I/F) 210, a display device 211, and an input device 212. The CPU 206, the RAM 207, the ROM 208, the HDD 209, the communication I/F 210, the display device 211, and the input device 212 are mutually connected through a bus 213. The display device 211 and the input device 212 may be connected to the bus 213 through a driving device (not shown) for driving those devices.

In Fig. 6C, respective units forming the information processing system 205 are illustrated as an integrated device, but some of those functions may be configured by an external device. For example, the display device 211 and the input device 212 may be external devices separate from a section constituting the functions of the computer including the CPU 206 and the like.

The CPU 206 performs predetermined operations in accordance with programs stored in the RAM 207, the HDD 209, and the like, and also has a function of controlling each unit of the information processing system 205. The RAM 207 is formed of a volatile storage medium, and provides a temporary memory area required for the operations of the CPU 206. The ROM 208 is formed of a non-volatile storage medium, and stores required information such as programs to be used for operations of the information processing system 205. The HDD 209 is formed of a non-volatile storage medium, and is a storage device that stores, for example, information regarding the state of vibration to be excited in the vibrating body 101.

The communication I/F 210 is a communication interface based on a standard such as Wi-Fi (trademark) or 5G, and is a module for performing communication to/from another device. The display device 211 is a liquid crystal display, an organic light emitting diode (OLED) display, or the like, and is used for displaying moving images, still images, characters, and the like. The input device 212 is a button, a touch panel, a keyboard, a pointing device, or the like, and is used by the user to operate the information processing system 205. The display device 211 and the input device 212 may be integrally formed as a touch panel.

The hardware configuration illustrated in Fig. 6C is illustrated as an example, and devices other than those devices may be added, and some of the devices may be omitted. Some of the devices may also be replaced by other devices having similar functions. Further, some of the functions may be provided by other devices through a network, and the functions constituting the cell detachment device according to the present disclosure may be implemented in a distributed manner across a plurality of devices. For example, the HDD 209 may be replaced by a solid state drive (SSD) using a semiconductor element, such as a flash memory, or may be replaced by a cloud storage.

The CPU 206 implements control of the supply unit 23 by loading a program stored in the ROM 208 or the like into the RAM 207 and executing the program. The CPU 206 also implements the function of the display unit 110 by controlling the display device 211. The CPU 206 also implements the function of the storage unit 111 by controlling the HDD 209.

The cell detachment method according to the present disclosure includes the following first to fourth embodiments. Each embodiment is described below.

<First Embodiment>

The present disclosure provides the following cell detachment method as the first embodiment.

This embodiment is a cell detachment method of detaching cells adhering to a culture surface of a culture substrate from the culture surface, and includes:

an arrangement step of arranging a first acoustic transmission medium and a second acoustic transmission medium between the culture substrate and a vibrating body; and

a detachment step of detaching the cells from the culture surface by vibrating the vibrating body under a state in which the culture substrate is in contact with the first acoustic transmission medium and the second acoustic transmission medium.

When the first embodiment of the present disclosure is carried out, a configuration in which the first and second acoustic transmission media are in contact with the culture substrate side is adopted.
A position at which the second acoustic transmission medium is arranged may change depending on a shape of a bottom surface of the culture substrate and a surface shape of the first acoustic transmission medium.
Configuration views of the acoustic transmission medium used in the cell detachment method which is the first embodiment of the present disclosure are illustrated in Fig. 1A to Fig. 1C.
Fig. 1A is a view of an arrangement image of a second acoustic transmission medium 132 in a combination of the culture substrate 21 having a flat substrate bottom surface and the first acoustic transmission medium 131 having a central portion that is convex.
Fig. 1B is a view of an arrangement image of the second acoustic transmission medium 132 in a combination of the general-purpose culture substrate 21 having a warped substrate bottom surface and the flat first acoustic transmission medium 131.
Fig. 1C is a view of an arrangement image of the second acoustic transmission medium 132 in a combination of the general-purpose culture substrate 21 having a warped substrate bottom surface and the flat first acoustic transmission medium 131.
As described above, the culture substrate 21 varies greatly among model numbers of different manufacturers and among production lots. In contrast, in this embodiment, the second acoustic transmission medium 132 is arranged on the culture substrate 21 side, and the first acoustic transmission medium 131 and the second acoustic transmission medium 132 complement each other to achieve close contact. Thus, even when there is such variation in the culture substrate, it is easy to apply appropriate vibration corresponding thereto.
In this embodiment, as illustrated in Fig. 1A to Fig. 1C, it is preferred that the first acoustic transmission medium 131 and the second acoustic transmission medium 132 be in contact with the culture substrate 21 side, and the vibrating body 101 be in contact with the first acoustic transmission medium 131.
Fig. 5B is a flow chart for illustrating an example of more detailed steps of the embodiment of the present disclosure.
First, as the arrangement step, the first acoustic transmission medium is placed on the vibrating body (Step S210).
After that, the second acoustic transmission medium is placed on the first acoustic transmission medium (Step S220).
Next, as the pressing step, the first and second acoustic transmission media are brought into contact with the culture substrate (Step S230).
After that, pressure is applied from above the culture substrate through use of a weight or the like (Step S240).
As the detachment step, ultrasonic application conditions are set (Step S250).
Next, ultrasonic application is started (Step S260).
Finally, the detached cells are collected (Step S270).

<Second Embodiment>

The present disclosure provides the following cell detachment method as the second embodiment.
This embodiment is a cell detachment method of detaching cells adhering to a culture surface of a culture substrate from the culture surface, and includes:

an arrangement step of arranging a first acoustic transmission medium and a second acoustic transmission medium between the culture substrate and a vibrating body; and
a detachment step of detaching the cells from the culture surface by vibrating the vibrating body under a state in which the vibrating body is in contact with the first acoustic transmission medium and the second acoustic transmission medium.

When the second embodiment of the present disclosure is carried out, a configuration in which the first and second acoustic transmission media are in contact with the vibrating body side is adopted.
The position at which the second acoustic transmission medium is arranged may change depending on the shape of the bottom surface of the culture substrate, a shape of an upper surface of the vibrating body, and the surface shape of the first acoustic transmission medium.
Configuration views of the acoustic transmission medium used in the cell detachment method which is the second embodiment of the present disclosure are illustrated in Fig. 2A to Fig. 2C.
Fig. 2A is a view of an arrangement image of the second acoustic transmission medium 132 in a combination of the culture substrate 21 having a flat substrate bottom surface, a vibrating body having a flat upper surface, and the first acoustic transmission medium 131 having a central portion that is convex downward.

Fig. 2B is a view of an arrangement image of the second acoustic transmission medium 132 in a combination of the general-purpose culture substrate 21 having a warped substrate bottom surface, the flat vibrating body, and the first acoustic transmission medium 131 having a central portion that is convex upward and convex downward.

Fig. 2C is a view of an arrangement image of the second acoustic transmission medium 132 in a combination of the general-purpose culture substrate 21 having a warped substrate bottom surface, the flat vibrating body, and the first acoustic transmission medium 131 having a central portion that is convex upward and concave downward.

In this embodiment, as illustrated in Fig. 2A to Fig. 2C, it is preferred that the first acoustic transmission medium 131 and the second acoustic transmission medium 132 be in contact with the vibrating body, and the culture substrate 21 be in contact with the first acoustic transmission medium.

<Third Embodiment>

The present disclosure provides the following cell detachment method as the third embodiment.

This embodiment is a cell detachment method of detaching cells adhering to a culture surface of a culture substrate from the culture surface, and includes:

an arrangement step of arranging a first acoustic transmission medium and a second acoustic transmission medium between the culture substrate and a vibrating body; and

a detachment step of detaching the cells from the culture surface by vibrating the vibrating body under a state in which the culture substrate is in contact with the first acoustic transmission medium and the second acoustic transmission medium and the vibrating body is in contact with the first acoustic transmission medium and the second acoustic transmission medium.

When the third embodiment of the present disclosure is carried out, a configuration in which the first and second acoustic transmission media are in contact with both the culture substrate and the vibrating body is adopted.

The position at which the second acoustic transmission medium is arranged may change depending on the shape of the bottom surface of the culture substrate and the surface shape of the first acoustic transmission medium.

Configuration views of the acoustic transmission medium used in the cell detachment method which is the third embodiment of the present disclosure are illustrated in Fig. 3A to Fig. 3C.

Fig. 3A is a view of an arrangement image of the second acoustic transmission medium 132 in a combination of the culture substrate 21 having a flat substrate bottom surface and the first acoustic transmission medium 131 having a central portion that is convex upward and convex downward.

Fig. 3B is a view of an arrangement image of the second acoustic transmission medium 132 in a combination of the general-purpose culture substrate 21 having a warped substrate bottom surface and the first acoustic transmission medium 131 having a central portion that is convex upward and convex downward.

Fig. 3C is a view of an arrangement image of the second acoustic transmission medium 132 in a combination of the general-purpose culture substrate 21 having a warped substrate bottom surface and the first acoustic transmission medium 131 having a central portion that is convex upward and concave downward.

<Fourth Embodiment>

The present disclosure provides the following cell detachment method as the fourth embodiment.

This embodiment is a cell detachment method of detaching cells adhering to a culture surface of a culture substrate from the culture surface, and includes:

an arrangement step of arranging a first acoustic transmission medium and a second acoustic transmission medium between the culture substrate and a vibrating body; and

a detachment step of detaching the cells from the culture surface by vibrating the vibrating body under a state in which the culture substrate is in contact with the first acoustic transmission medium and the second acoustic transmission medium, the vibrating body is in contact with the first acoustic transmission medium and the second acoustic transmission medium, and the second acoustic transmission medium is continuously arranged in the vertical direction.

When the fourth embodiment of the present disclosure is carried out, a configuration in which the first and second acoustic transmission media are in contact with both the culture substrate and the vibrating body is adopted.

The position at which the second acoustic transmission medium is arranged may change depending on the shape of the bottom surface of the culture substrate and the surface shape of the first acoustic transmission medium.

Configuration views of the acoustic transmission medium used in the cell detachment method which is the fourth embodiment of the present disclosure are illustrated in Fig. 4A to Fig. 4C.

Fig. 4A is a view of an arrangement image of the second acoustic transmission medium 132 in a combination of the culture substrate 21 having a flat substrate bottom surface and the first acoustic transmission medium 131 divided into a central portion and an outer peripheral portion, the central portion and the outer peripheral portion both being flat.

Fig. 4B is a view of an arrangement image of the second acoustic transmission medium 132 in a combination of the general-purpose culture substrate 21 having a warped substrate bottom surface and the first acoustic transmission medium 131 divided into a central portion and an outer peripheral portion, the central portion being flat, the outer peripheral portion becoming lower toward an outer edge.

Fig. 4C is a view of an arrangement image of the second acoustic transmission medium 132 in a combination of the general-purpose culture substrate 21 having a warped substrate bottom surface and the first acoustic transmission medium 131 divided into a central portion and an outer peripheral portion, the central portion being flat and having a diameter different from that of Fig. 4B, the outer peripheral portion becoming lower toward the outer edge and having a width different from that of Fig. 4B, as well as a top view of the arrangement image.

When the antinode in the vibration pattern of the vibrating body appears near the center of the vibrating body, it is possible to achieve efficient detachment by, as in this embodiment, providing the first acoustic transmission medium 131 in the central portion and ensuring close contact between the culture substrate and the acoustic transmission medium.

[Examples]

(Example 1)

Examples 1 to 28, 32, and 33 correspond to the first embodiment.

In Example 1, the configuration of the acoustic transmission medium illustrated in the schematic view of Fig. 1A was used. A Nunc (trademark) Φ35-mm dish manufactured by Thermo Fisher Scientific Inc. was used as the culture substrate. Glass having an outer diameter of 70 mm and a thickness of 4 mm was used as the vibrating plate of the cell detachment device. PZT was used for the material composition of the piezoelectric body, and the piezoelectric body was set to have an outer diameter of 70 mm, which is the same as that of the vibrating plate, an inner diameter of 57 mm, and a thickness of 2 mm in consideration of the neutral plane being on the vibrating plate side. The cell detachment device was configured as in Fig. 6A, and a silicone rubber having a hardness of 30° in a center-convex disc shape having a central height (1) of 0.9 mm, a side height (2) of 0.4 mm, and a diameter (3) of 32 mm, as illustrated in the schematic view of Fig. 9A, was placed on the vibrating plate as the first acoustic transmission medium so as to have the center aligned with that of the vibrating plate. This silicone rubber was set as the first acoustic transmission medium. The tanδ of this silicone rubber obtained by dynamic viscoelasticity measurement at room temperature when a vibration having a frequency of 1 Hz was applied thereto was 0.13. Water serving as the second acoustic transmission medium was dropped with the dispenser in an amount of 200 μL onto the central portion of the first acoustic transmission medium. Next, the Nunc (trademark) Φ35-mm dish manufactured by Thermo Fisher Scientific Inc., serving as the culture substrate on which cells were cultured, was slowly placed on the vibrating plate with the centers of both aligned. After that, a weight of 100 g was placed on the lid of the culture substrate to apply pressure. The schematic view for illustrating the configuration of the acoustic transmission medium after the application of pressure is Fig. 1B. In regard to the contact areas of the first and second acoustic transmission media with the culture substrate, the water serving as the second acoustic transmission medium was colored with food coloring, it was confirmed from a photograph taken from above after the application of pressure that there was no air layer between the acoustic transmission medium and the culture substrate, and then the respective contact areas of the first and second acoustic transmission media were calculated. The contact area ratio of the first acoustic transmission medium was 10%. Further, the contact angle between the first acoustic transmission medium and the second acoustic transmission medium was 110°.

For cell culturing, Chinese hamster ovary (CHO) cells were seeded in the Nunc (trademark) Φ35-mm dish manufactured by Thermo Fisher Scientific Inc. at a density of 10,000 cells/$cm^2$ and cultured in an environment of 37°C and a $CO_2$ concentration of 5%. The medium used was Ham's F12 (manufactured by Thermo Fisher Scientific Inc.) with 10% fetal bovine serum (manufactured by Sigma-Aldrich Co. LLC) and 1% penicillin-streptomycin (10,000 U/ml; manufactured by Thermo Fisher Scientific Inc.) added. Culturing was performed for 48 hours, and the state of the cells was observed with a phase-contrast microscope to examine cell adhesion and proliferation. The cell occupancy ratio of the dish was about 80%.

(Cell Detachment)

The medium in the dish was removed, and the cells were washed with PBS(-), and then immersed in PBS(-) serving as the detachment liquid for 3 minutes.

After that, the culture substrate was placed in the cell detachment device at 25°C, and cell detachment was performed for 2 minutes with a frequency sweep vibration (a frequency of from 22 kHz to 27 kHz, a frequency sweep period of 1 s, and a voltage of 90 V) in the standing wave mode at an environmental temperature of 25°C. In this case, a standing wave was generated by applying an AC voltage with a 180-degree phase difference to the negatively poled electrodes (A2 and B2) with respect to the positively poled electrodes (A1, A3, B1, and B3) among the electrodes of Fig. 10. The vibration mode due to such driving is referred to as "Vibration Mode A" in Fig. 12 described later.

After the detached cells were collected, all cells that failed to be detached by ultrasound were detached from the dish after ultrasonic detachment through use of a cell scraper, and the number of cells was measured with a hemocytometer. The total number of detached cells was defined as a sum of the number of cells detached by ultrasound and the number of cells detached by the cell scraper after the ultrasonic detachment, and the detachment rate was defined as a ratio of the number of ultrasonically detached cells to the total number of detached cells, to thereby calculate a value of the detachment rate. For the variation, the detachment rate was determined at n=10, and the standard deviation of the detachment rate was evaluated as A for less than 5%, B for 5% or more and less than 7.5%, C for 7.5% or more and less than 10%, and D for 10% or more. Results of the evaluation in Example 1 were a detachment rate of 97.4% and a variation evaluation of A.

(Example 2)

Example 2 was carried out under the same conditions as in Example 1, except that the first acoustic transmission medium was changed as described below and the amount of the second acoustic transmission medium used was changed as described below. That is, a silicone rubber having a hardness of 30° in a disc shape having a central height (1) =side height (2) of 0.9 mm and a diameter (3) of 32 mm, as illustrated in the schematic view of Fig. 9B, was used as the first acoustic transmission medium on the vibrating plate. As the second acoustic transmission medium, water was placed by being sprayed with a spray device in an amount of 70 μL uniformly onto the first acoustic transmission medium. The contact area ratio of the first acoustic transmission medium was 20%.

Results of the evaluation in Example 2 were a detachment rate of 90.3% and a variation evaluation of C.

(Example 3)

Example 3 was carried out under the same conditions as in Example 1, except that the cell detachment conditions were changed as described below. Cell detachment was performed under the cell detachment conditions involving a frequency sweep vibration (a frequency of from 67.5 kHz to 72.5 kHz, a frequency sweep period of 1 s, and a voltage of 90 V) in the standing wave mode for 2 minutes. Although the frequency band was different from that of Example 1, a standing wave was generated under the same condition for applying an AC voltage with a 180-degree phase difference to the negatively poled electrodes (A2 and B2) with respect to the positively poled electrodes (A1, A3, B1, and B3). The vibration mode due to such driving is referred to as "Vibration Mode B" in Fig. 12 described later.

Results of the evaluation in Example 3 were a detachment rate of 90.0% and a variation evaluation of C.

(Example 4)

Example 4 was carried out under the same conditions as in Example 2, except that the cell detachment conditions were changed as described below. Cell detachment was performed under the cell detachment conditions involving a frequency sweep vibration (a frequency of from 67.5 kHz to 72.5 kHz, a frequency sweep period of 1 s, and a voltage of 90 V) in the standing wave mode for 2 minutes. Results of the evaluation in Example 4 were a detachment rate of 96.8% and a variation evaluation of A.

(Example 5)

Example 5 was carried out under the same conditions as in Example 1, except that the first acoustic transmission medium was changed as described below. That is, a silicone rubber having a hardness of 30° in a modified center-convex disc shape having a central height (1) of 0.9 mm, an upper surface height (1)' of 0.3 mm, a side height (2) of 0.3 mm, a diameter (3) of 32 mm, and an inner diameter (3)' of 5 mm, as illustrated in the schematic view of Fig. 9C, was used as the first acoustic transmission medium on the vibrating plate. Water serving as the second acoustic transmission medium was dropped with the dispenser in an amount of 250 μL onto the first acoustic transmission medium. The contact area ratio of the first acoustic transmission medium was 5%.

Results of the evaluation in Example 5 were a detachment rate of 90.2% and a variation evaluation of B.

(Example 6)

Example 6 was carried out under the same conditions as in Example 1, except that the first acoustic transmission medium was changed as described below. That is, a silicone rubber having a hardness of 30° in a modified disc shape having a central height (1) of 0.9 mm, a side height (2) of 0.7 mm, a side height (2)' of 0.4 mm, a diameter (3) of 32 mm, and an inner diameter (3)' of 20 mm, as illustrated in the schematic view of Fig. 9D, was used as the first acoustic transmission medium on the vibrating plate. Water serving as the second acoustic transmission medium was dropped with the dispenser in an amount of 200 μL onto the first acoustic transmission medium. The contact area ratio of the first acoustic transmission medium was 30%.

Results of the evaluation in Example 6 were a detachment rate of 98.2% and a variation evaluation of A.

(Example 7)

Example 7 was carried out under the same conditions as in Example 1, except that the second acoustic transmission medium was changed to a PVA gel and the amount used was changed.

The PVA gel was prepared by the following method. Denka Poval (trademark) K-24E (manufactured by Denka Company Limited) was weighed so as to achieve a concentration of 3.7 mass%, and pure water was added thereto to prepare a mixture having a total volume of 80 mL. The resultant mixture was stirred while being heated in a thermostatic chamber, and the PVA was completely dissolved to obtain a PVA aqueous solution. Next, borax (sodium tetraborate decahydrate (special grade); manufactured by Kishida Chemical Co., Ltd.) was weighed, and pure water was added thereto to prepare an aqueous solution having a concentration of 1.0 mass% and a total volume of 16 mL. While the PVA aqueous solution was being stirred, the borax aqueous solution was gradually dropped thereonto, and the mixture was stirred until uniformity was achieved, to thereby obtain the PVA gel. The tanδ obtained by dynamic viscoelasticity measurement at room temperature when a vibration having a frequency of 0.1 Hz was applied to this PVA gel was 1.7, and the PVA gel can be regarded as a solid with flowability. The tanδ obtained by dynamic viscoelasticity measurement at room temperature when a vibration having a frequency of 1 Hz was applied thereto was 0.856.

0.2 Grams of the PVA gel serving as the second acoustic transmission medium was weighed, spread thinly into a circular sheet shape having a diameter of 25 mm, and placed on the first acoustic transmission medium. Next, the Nunc (trademark) Φ35-mm dish manufactured by Thermo Fisher Scientific Inc., serving as the culture substrate on which cells were cultured, was slowly placed on the vibrating plate with the centers of both aligned. After that, a weight of 100 g was placed on the lid of the culture substrate to apply pressure.

The contact area ratio of the first acoustic transmission medium was 10%.

Results of the evaluation in Example 7 were a detachment rate of 85.7% and a variation evaluation of C.

(Example 8)

Example 8 was carried out under the same conditions as in Example 1, except that the second acoustic transmission medium was changed to an ultrasound gel and the amount used was changed.

As the second acoustic transmission medium, 0.2 g of the ultrasound gel (Pro Jelly for Ultrasonic Examination, Normal Type; manufactured by JEX Co., Ltd.) was weighed, and placed by being spread uniformly within a range of 20 mm from the center of the first acoustic transmission medium. Next, the Nunc (trademark) Φ35-mm dish manufactured by Thermo Fisher Scientific Inc., serving as the culture substrate on which cells were cultured, was slowly placed on the vibrating plate with the centers of both aligned. After that, a weight of 100 g was placed on the lid of the culture substrate to apply pressure.

The contact area ratio of the first acoustic transmission medium was 10%.

Results of the evaluation in Example 8 were a detachment rate of 96.4% and a variation evaluation of A.

(Example 9)

Example 9 was carried out under the same conditions as in Example 1, except that the second acoustic transmission medium was changed to glycerin and the amount used was changed.

0.27 Grams of glycerin (glycerin (special grade); manufactured by Kishida Chemical Co., Ltd.) serving as the second acoustic transmission medium was weighed, and placed by being spread uniformly onto the first acoustic transmission medium. Next, the Nunc (trademark) Φ35-mm dish manufactured by Thermo Fisher Scientific Inc., serving as the culture substrate on which cells were cultured, was slowly placed on the vibrating plate with the centers of both aligned. After that, a weight of 100 g was placed on the lid of the culture substrate to apply pressure.

The contact area ratio of the first acoustic transmission medium was 10%. Further, the contact angle between the first acoustic transmission medium and the second acoustic transmission medium was 100°.

Results of the evaluation in Example 9 were a detachment rate of 94.3% and a variation evaluation of A.

(Example 10)

Example 10 was carried out under the same conditions as in Example 1, except that the first acoustic transmission medium was changed as described below. That is, a silicone rubber having a hardness of 30° in a center-convex disc shape having a central height (1) of 0.9 mm, a side height (2) of 0.4 mm, and a diameter (3) of 32 mm, as illustrated in the schematic view of Fig. 9A, was used as the first acoustic transmission medium on the vibrating plate, the silicone rubber being provided, on the surface on the culture substrate side, with 12 straight grooves having a width of 0.3 mm and a depth of 0.2 mm, radially from the center, at 30° intervals. Water serving as the second acoustic transmission medium was dropped with the dispenser in an amount of 1,400 μL onto the first acoustic transmission medium. The contact area ratio of the first acoustic transmission medium was 10%.

Results of the evaluation in Example 10 were a detachment rate of 92.1% and a variation evaluation of A.

(Example 11)

Example 11 was carried out under the same conditions as in Example 1, except that the second acoustic transmission medium was changed to ethanol and the amount used was changed.

Ethanol (ethanol (special grade); manufactured by Kishida Chemical Co., Ltd.) serving as the second acoustic transmission medium was placed by being dropped with the dispenser in an amount of 1,400 μL onto the central portion of the first acoustic transmission medium. Next, the Nunc (trademark) Φ35-mm dish manufactured by Thermo Fisher Scientific Inc., serving as the culture substrate on which cells were cultured, was slowly placed on the vibrating plate with the centers of both aligned. After that, a weight of 100 g was placed on the lid of the culture substrate to apply pressure.

The contact area ratio of the first acoustic transmission medium was 10%. Further, the contact angle between the first acoustic transmission medium and the second acoustic transmission medium was 40°.

Results of the evaluation in Example 11 were a detachment rate of 94.0% and a variation evaluation of A.

(Example 12)

Example 12 was carried out under the same conditions as in Example 1, except that the second acoustic transmission medium was changed to a 70% ethanol aqueous solution and the amount used was changed.

The 70% ethanol aqueous solution serving as the second acoustic transmission medium was obtained by mixing ethanol (ethanol (special grade); manufactured by Kishida Chemical Co., Ltd.) and pure water at a volume ratio of 70:30. The obtained 70% ethanol aqueous solution was placed by being dropped with the dispenser in an amount of 1,400 μL onto the central portion of the first acoustic transmission medium. Next, the Nunc (trademark) Φ35-mm dish manufactured by Thermo Fisher Scientific Inc., serving as the culture substrate on which cells were cultured, was slowly placed on the vibrating plate with the centers of both aligned. After that, a weight of 100 g was placed on the lid of the culture substrate to apply pressure.

The contact area ratio of the first acoustic transmission medium was 10%. Further, the contact angle between the first acoustic transmission medium and the second acoustic transmission medium was 55°.

Results of the evaluation in Example 12 were a detachment rate of 94.6% and a variation evaluation of A.

(Example 13)

Example 13 was carried out under the same conditions as in Example 1, except that the second acoustic transmission medium was changed to a surfactant aqueous solution 1 (water+surfactant 1) and the amount used was changed.

The surfactant aqueous solution serving as the second acoustic transmission medium was obtained by mixing sodium lauryl sulfate (sodium lauryl sulfate (1st grade); manufactured by Kishida Chemical Co., Ltd.) in pure water to prepare a 10-mmol/L solution. The obtained surfactant aqueous solution 1 (sodium lauryl sulfate aqueous solution) was placed by being dropped with the dispenser in an amount of 1,400 μL onto the central portion of the first acoustic transmission medium. Next, the Nunc (trademark) Φ35-mm dish manufactured by Thermo Fisher Scientific Inc., serving as the culture substrate on which cells were cultured, was slowly placed on the vibrating plate with the centers of both aligned. After that, a weight of 100 g was placed on the lid of the culture substrate to apply pressure.

The contact area ratio of the first acoustic transmission medium was 10%. Further, the contact angle between the first acoustic transmission medium and the second acoustic transmission medium was 50°.

Results of the evaluation in Example 13 were a detachment rate of 93.9% and a variation evaluation of A.

(Example 14)

Example 14 was carried out under the same conditions as in Example 1, except that the second acoustic transmission medium was changed to a surfactant aqueous solution 2 (water+surfactant 2) and the amount used was changed.
The surfactant aqueous solution serving as the second acoustic transmission medium was obtained by mixing Pluronic (trademark) F-68 (Pluronic (trademark) F-68 Non-ionic Surfactant (100X); manufactured by Thermo Fisher Scientific Inc.) in pure water to prepare a 0.1-mass% solid-content solution. The obtained surfactant aqueous solution 2 was placed by being dropped with the dispenser in an amount of 1,400 μL onto the central portion of the first acoustic transmission medium. Next, the Nunc (trademark) Φ35-mm dish manufactured by Thermo Fisher Scientific Inc., serving as the culture substrate on which cells were cultured, was slowly placed on the vibrating plate with the centers of both aligned. After that, a weight of 100 g was placed on the lid of the culture substrate to apply pressure.
The contact area ratio of the first acoustic transmission medium was 10%. Further, the contact angle between the first acoustic transmission medium and the second acoustic transmission medium was 75°.
Results of the evaluation in Example 14 were a detachment rate of 93.2% and a variation evaluation of A.

(Example 15)

Example 15 was carried out under the same conditions as in Example 1, except that the first acoustic transmission medium was changed as described below. That is, a silicone rubber having a hardness of 30° in a center-convex disc shape having a central height (1) of 0.9 mm, a side height (2) of 0.4 mm, and a diameter (3) of 32 mm (schematic view of Fig. 9A) which was used in Example 1 was used as the first acoustic transmission medium on the vibrating plate, the silicone rubber having been subjected to hydrophilization treatment on the convex-shaped surface. Water serving as the second acoustic transmission medium was dropped onto the surface of the silicone rubber that had been subjected to hydrophilization treatment, and the contact angle was measured to be 25°.
As the second acoustic transmission medium, water was placed by being dropped with the dispenser in an amount of 1,400 μL onto the central portion of the first acoustic transmission medium. The contact area ratio of the first acoustic transmission medium was 10%.
Results of the evaluation in Example 15 were a detachment rate of 95.5% and a variation evaluation of A.

(Example 16)

Example 16 was carried out under the same conditions as in Example 1, except that assembly was performed by changing the vibrating plate 11 in the device configuration view of Fig. 6A to glass that had been subjected to hydrophilization treatment on the upper surface.
Water serving as the second acoustic transmission medium was dropped onto the surface of the glass that had been subjected to hydrophilization treatment, and the contact angle was measured to be 20°.
As the second acoustic transmission medium, water was placed by being dropped with the dispenser in an amount of 1,400 μL onto the central portion of the first acoustic transmission medium. The contact area ratio of the first acoustic transmission medium was 10%.
Results of the evaluation in Example 16 were a detachment rate of 95.1% and a variation evaluation of A.

(Example 17)

Example 17 was carried out under the same conditions as in Example 1, except that the culture substrate 21 in the device configuration view of Fig. 6A was changed to a culture substrate that had been subjected to hydrophilization treatment on the outer surface including the side surface and the bottom surface.
Water serving as the second acoustic transmission medium was dropped onto the bottom surface of the culture substrate that had been subjected to hydrophilization treatment, and the contact angle was measured to be 25°.
As the second acoustic transmission medium, water was placed by being dropped with the dispenser in an amount of 1,400 μL onto the central portion of the first acoustic transmission medium. The contact area ratio of the first acoustic transmission medium was 10%.
Results of the evaluation in Example 17 were a detachment rate of 94.8% and a variation evaluation of A.

(Example 18)

Example 18 was carried out under the same conditions as in Example 1, except that assembly was performed by changing the vibrating plate 11 to protruding-shaped glass as in the device configuration view of Fig. 6B.

The protruding-shaped glass used was obtained by bonding glass having an outer diameter of 32 mm and a thickness of 2 mm to glass having an outer diameter of 70 mm and a thickness of 4 mm.
As the second acoustic transmission medium, water was placed by being dropped with the dispenser in an amount of 1,400 μL onto the central portion of the first acoustic transmission medium. The contact area ratio of the first acoustic transmission medium was 10%.
Results of the evaluation in Example 18 were a detachment rate of 95.8% and a variation evaluation of A.

(Example 19)

Example 19 was carried out under the same conditions as in Example 18, except that the second acoustic transmission medium was changed to a 70% ethanol aqueous solution.
That is, as the second acoustic transmission medium, a 70% ethanol aqueous solution was placed by being dropped with the dispenser in an amount of 1,400 μL onto the central portion of the first acoustic transmission medium. The contact area ratio of the first acoustic transmission medium was 10%.
Results of the evaluation in Example 19 were a detachment rate of 97.4% and a variation evaluation of A.

(Example 20)

Example 20 was carried out under the same conditions as in Example 1, except that the second acoustic transmission medium was changed to a 70% ethanol aqueous solution and the amount used was also changed.
That is, the same 70% ethanol aqueous solution as that used in Example 12 was used as the second acoustic transmission medium. The 70% ethanol aqueous solution was placed by being dropped with the dispenser in a liquid amount of 1,400 μL onto the central portion of the first acoustic transmission medium. The contact area ratio of the first acoustic transmission medium was 10%.
Results of the evaluation in Example 20 were a detachment rate of 97.1% and a variation evaluation of A.

(Example 21)

Example 21 was carried out under the same conditions as in Example 1, except that the amount of water used as the second acoustic transmission medium was changed.
That is, as the second acoustic transmission medium, water was placed by being dropped with the dispenser in an amount of 1,200 μL onto the central portion of the first acoustic transmission medium. The contact area ratio of the first acoustic transmission medium was 10%.
Results of the evaluation in Example 21 were a detachment rate of 91.1% and a variation evaluation of B.

(Example 22)

Example 22 was carried out under the same conditions as in Example 2, except that the amount of water used as the second acoustic transmission medium was changed.
That is, as the second acoustic transmission medium, water was placed by being sprayed with the spray device in an amount of 40 μL uniformly onto the first acoustic transmission medium. The contact area ratio of the first acoustic transmission medium was 20%.
Results of the evaluation in Example 22 were a detachment rate of 90.2% and a variation evaluation of C.

(Example 23)

Example 23 was carried out under the same conditions as in Example 1, except that the amount of water used as the second acoustic transmission medium was changed.
That is, as the second acoustic transmission medium, water was placed by being dropped with the dispenser in an amount of 1,400 μL onto the central portion of the first acoustic transmission medium. The contact area ratio of the first acoustic transmission medium was 10%.
Results of the evaluation in Example 23 were a detachment rate of 90.4% and a variation evaluation of B.

(Example 24)

Example 24 was carried out under the same conditions as in Example 2, except that the amount of water used as the second acoustic transmission medium was changed.
That is, as the second acoustic transmission medium, water was placed by being sprayed with the spray device in an amount of 35 μL uniformly onto the first acoustic transmission medium. The contact area ratio of the first acoustic transmission medium was 20%. It was also confirmed that the second acoustic transmission medium did not spread over the entire surface, and 10% of the total area was an air layer.

Results of the evaluation in Example 24 were a detachment rate of 84.2% and a variation evaluation of C.

(Example 25)

Example 25 was carried out under the same conditions as in Example 5, except that the amount of water used as the second acoustic transmission medium was changed.

That is, as the second acoustic transmission medium, water was placed by being sprayed with the spray device in an amount of 25 μL uniformly onto the first acoustic transmission medium. The contact area ratio of the first acoustic transmission medium was 10%. It was also confirmed that the second acoustic transmission medium did not spread over the entire surface, and 20% of the total area was an air layer.

Results of the evaluation in Example 25 were a detachment rate of 83.5% and a variation evaluation of C.

(Example 26)

Example 26 was carried out under the same conditions as in Example 1, except that a urethane rubber with a center-convex disc shape having a central height (1) of 0.9 mm, a side height (2) of 0.4 mm, and a diameter (3) of 32 mm (schematic view of Fig. 9A) which was used in Example 1 was used as the first acoustic transmission medium on the vibrating plate. The tanδ of this urethane rubber, obtained by performing viscoelasticity measurement at room temperature when a vibration having a frequency of 1 Hz was applied thereto was 0.23.

As the second acoustic transmission medium, water was placed by being dropped with the dispenser in an amount of 200 μL onto the central portion of the first acoustic transmission medium. The contact area ratio of the first acoustic transmission medium was 10%. Further, the contact angle between the first acoustic transmission medium and the second acoustic transmission medium was 80°.

Results of the evaluation in Example 26 were a detachment rate of 90.1% and a variation evaluation of B.

(Example 27)

Example 27 was carried out under the same conditions as in Example 1, and a silicone rubber having a hardness of 10° in a center-convex disc shape having a central height (1) of 0.9 mm, a side height (2) of 0.4 mm, and a diameter (3) of 32 mm (schematic view of Fig. 9A) which was used in Example 1 was used as the first acoustic transmission medium on the vibrating plate. The tanδ of this silicone rubber obtained by performing viscoelasticity measurement at room temperature when a vibration having a frequency of 1 Hz was applied thereto was 0.14.

As the second acoustic transmission medium, water was placed by being dropped with the dispenser in an amount of 200 μL onto the central portion of the first acoustic transmission medium. The contact area ratio of the first acoustic transmission medium was 10%. Further, the contact angle between the first acoustic transmission medium and the second acoustic transmission medium was 110°.

Results of the evaluation in Example 27 were a detachment rate of 93.3% and a variation evaluation of B.

(Example 28)

Example 28 was carried out under the same conditions as in Example 1, except that a silicone rubber having a hardness of 50° in a center-convex disc shape having a central height (1) of 0.9 mm, a side height (2) of 0.4 mm, and a diameter (3) of 32 mm (schematic view of Fig. 9A) which was used in Example 1 was used as the first acoustic transmission medium on the vibrating plate. The tanδ of this silicone rubber obtained by performing viscoelasticity measurement at room temperature when a vibration having a frequency of 1 Hz was applied thereto was 0.05.

As the second acoustic transmission medium, water was placed by being dropped with the dispenser in an amount of 200 μL onto the central portion of the first acoustic transmission medium. The contact area ratio of the first acoustic transmission medium was 10%. Further, the contact angle between the first acoustic transmission medium and the second acoustic transmission medium was 110°.

Results of the evaluation in Example 28 were a detachment rate of 96.4% and a variation evaluation of A.

(Example 29)

Example 29 corresponds to the second embodiment.

Example 29 was carried out under the same conditions as in Example 1, except that the steps during the placement were changed and the configuration of the acoustic transmission medium illustrated in the schematic view of Fig. 2A was used.

The Nunc (trademark) Φ35-mm dish manufactured by Thermo Fisher Scientific Inc., serving as the culture substrate on which cells were cultured, was brought, from a bottom surface or back side thereof, into contact with the convex surface of the first acoustic transmission medium similar to that in Example 1 with the centers aligned, and the entire surfaces were brought into close contact with each other so that no air layer was introduced. Next, water serving as the second acoustic transmission medium was dropped with the dispenser in an amount of 200 μL onto the central portion of the vibrating plate, and the Φ35-mm dish with the bottom surface in close contact with the first acoustic transmission medium was slowly placed on the vibrating plate with the centers of both aligned. After that, a weight of 100 g was placed on the lid of the culture substrate to apply pressure. The schematic view for illustrating the configuration of the acoustic transmission medium after the application of pressure is Fig. 2C. In regard to the contact areas of the first and second acoustic transmission media with the culture substrate, the water serving as the second acoustic transmission medium was colored with food coloring, it was confirmed from a photograph taken from below the vibrating plate after the application of pressure that there was no air layer between the acoustic transmission medium and the culture substrate, and then the respective contact areas of the first and second acoustic transmission media were calculated. The contact area ratio of the first acoustic transmission medium was 10%.

The cell detachment conditions and the detachment cell inspection were performed by the same method as in Example 1.

Results of the evaluation in Example 29 were a detachment rate of 97.2% and a variation evaluation of A.

(Example 30)

Example 30 corresponds to the third embodiment.

In Example 30, a Φ35-mm dish manufactured by Coming Incorporated was used as the culture substrate. A silicone rubber having a hardness of 10° having a central height (1) of 1.0 mm, a side height (2) of 0.3 mm, a diameter (3) of 32 mm, an upper surface height (4) of 0.4 mm, and a lower surface height (5) of 0.3 mm, as illustrated in the schematic view of Fig. 9E, was prepared as the first acoustic transmission medium. The cell detachment device was assembled, and water serving as the second acoustic transmission medium was dropped with the dispenser in an amount of 100 μL onto the central portion of the vibrating plate. Next, the silicone rubber serving as the first acoustic transmission medium was slowly placed on the vibrating plate with the lower surface of the silicone rubber aligned with the center of the vibrating plate. Further, water serving as the second acoustic transmission medium was dropped with the dispenser in an amount of 150 μL onto the central portion of the surface of the first acoustic transmission medium. The Φ35-mm dish manufactured by Corning Incorporated, serving as the culture substrate on which cells were cultured, was slowly placed on the vibrating plate with the centers of both aligned. After that, a weight of 100 g was placed on the lid of the culture substrate to apply pressure. The schematic view for illustrating the configuration of the acoustic transmission medium after the application of pressure is Fig. 3B. In regard to the contact areas of the first and second acoustic transmission media with the culture substrate, the water serving as the second acoustic transmission medium was colored with food coloring, it was confirmed from photographs taken from above and below after the application of pressure that there was no air layer between the acoustic transmission medium and the culture substrate, and then the respective contact areas of the upper surface and the lower surface of the first and second acoustic transmission media were calculated. The contact area ratio of the first acoustic transmission medium was 10% on the upper surface and 50% on the lower surface.

The cell detachment conditions and the detachment cell inspection were performed by the same method as in Example 1.

Results of the evaluation in Example 30 were a detachment rate of 94.4% and a variation evaluation of B.

(Example 31)

Example 31 corresponds to the fourth embodiment.

In Example 31, a ΦIWAKI 35-mm dish manufactured by AGC Techno Glass Co., Ltd. was used as the culture substrate. A silicone rubber having a hardness of 10° in a circular shape having a thickness (6) of 0.6 mm and a diameter (7) of 3.0 mm and a ring shape having a thickness (6)' of 0.6 mm, a width (8)' of 4.0 mm, and a diameter (7)' of 32 mm, as illustrated in the schematic view of Fig. 9F, was prepared as the first acoustic transmission medium. The cell detachment device was configured as in Fig. 4C, and the circular-shaped and ring-shaped silicone rubber serving as the first acoustic transmission medium was placed on the central portion of the vibrating plate.

Further, water serving as the second acoustic transmission medium was dropped with the dispenser in an amount of 400 μL onto the central portion of the surface of the first acoustic transmission medium. The ΦIWAKI Φ35-mm dish manufactured by AGC Techno Glass Co., Ltd., serving as the culture substrate on which cells were cultured, was slowly placed on the vibrating plate with the centers of both aligned. After that, a weight of 100 g was placed on the lid of the culture substrate to apply pressure. The schematic view for illustrating the configuration of the acoustic transmission medium after the application of pressure is Fig. 4C. In regard to the contact area of the first acoustic transmission medium with the culture substrate, the water serving as the second acoustic transmission medium was colored with food coloring, it was confirmed from a photograph taken from above after the application of pressure that there was no air layer between the acoustic transmission medium and the culture substrate, and then the contact area of the upper surface of the first acoustic transmission medium was calculated. The contact area ratio of the first acoustic transmission medium was 30% in total.

The cell detachment conditions and the detachment cell inspection were performed by the same method as in Example 1.

Results of the evaluation in Example 31 were a detachment rate of 92.3% and a variation evaluation of C.

(Example 32)

Example 32 was carried out under the same conditions as in Example 1, except that the cell detachment conditions were changed as described below. Cell detachment was performed under the cell detachment conditions involving a frequency sweep vibration (a frequency of from 67.5 kHz to 72.5 kHz, a frequency sweep period of 1 s, and a voltage of 90 V) in the above-mentioned traveling wave mode for 2 minutes. An AC voltage with a 90-degree phase difference between the A phase and the B phase of Fig. 10 was applied to generate a two-phase-driven traveling wave. The vibration mode due to such driving is referred to as "Vibration Mode C" in Fig. 12 described later.

Results of the evaluation in Example 32 were a detachment rate of 99.4% and a variation evaluation of A.

(Example 33)

Example 33 was carried out under the same conditions as in Example 1, except that the pressing step of placing the weight used for pressing in Example 1 was omitted and the amount of water serving as the second acoustic transmission medium was reduced.

That is, after the same first acoustic transmission medium as in Example 1 was placed so as to have the center aligned with that of the vibrating plate, water serving as the second acoustic transmission medium was dropped with the dispenser in an amount of 160 μL onto the central portion of the first acoustic transmission medium. The Nunc (trademark) Φ35-mm dish manufactured by Thermo Fisher Scientific Inc., serving as the culture substrate on which cells were cultured, was slowly placed on the vibrating plate with the centers of both aligned. The contact area ratio of the first acoustic transmission medium was 3%.

Results of the evaluation in Example 33 were a detachment rate of 84.1% and a variation evaluation of C.

(Comparative Examples)

(Comparative Example 1)

Comparative Example 1 was carried out under the same conditions as in Example 5, except that the second acoustic transmission medium was not used.

The same modified center-convex disc-shaped silicone rubber having a hardness of 30° as in Example 5, as illustrated in the schematic view of Fig. 9C, was placed as the first acoustic transmission medium so as to have the center aligned with that of the vibrating plate. Next, the Nunc (trademark) Φ35-mm dish manufactured by Thermo Fisher Scientific Inc., serving as the culture substrate on which cells were cultured, was slowly placed on the vibrating plate with the centers of both aligned. After that, a weight of 100 g was placed on the lid of the culture substrate to apply pressure. From a photograph taken from above after the application of pressure, an air layer was confirmed around the contact portion of the first acoustic transmission medium.

The contact area between the first acoustic transmission medium and the culture substrate was calculated in the same manner as in Example 1 and was 5%.

Results of the evaluation in Comparative Example 1 were a detachment rate of 81.2% and a variation evaluation of D.

(Comparative Example 2)

Comparative Example 2 was carried out under the same conditions as in Comparative Example 1, except that cell detachment was performed under the cell detachment conditions involving a frequency sweep vibration (a frequency of from 67.5 kHz to 72.5 kHz, a frequency sweep period of 1 s, and a voltage of 90 V) in the standing wave mode for 2 minutes.

From a photograph of the culture substrate taken from above after the application of pressure, an air layer was confirmed around the contact portion of the first acoustic transmission medium in the same manner as in Comparative Example 1.

The contact area between the first acoustic transmission medium and the culture substrate was calculated in the same manner as in Example 1 and was 5%.

Results of the evaluation in Comparative Example 2 were a detachment rate of 70.8% and a variation evaluation of D.

(Comparative Example 3)

Comparative Example 3 was carried out under the same conditions as in Example 1, but neither the culture substrate nor the vibrating body was in contact with the first acoustic transmission medium.

That is, in the same manner as in Example 1, the silicone rubber having a hardness of 30° in a center-convex disc shape having a central height (1) of 0.9 mm, a side height (2) of 0.4 mm, and a diameter (3) of 32 mm, as illustrated in the schematic view of Fig. 9A, was placed as the first acoustic transmission medium on the central portion of the vibrating plate. Next, water serving as the second acoustic transmission medium was dropped with the dispenser in equal portions of 50 μL each for a total of 300 μL. Next, the Nunc (trademark) Φ35-mm dish manufactured by Thermo Fisher Scientific Inc., serving as the culture substrate on which cells were cultured, was slowly placed on the vibrating plate with the centers of both aligned, and it was confirmed from a photograph taken from above the culture substrate that there was no contact portion of the first acoustic transmission medium and there was no air layer between the culture substrate and the second acoustic transmission medium.

The contact area between the first acoustic transmission medium and the culture substrate was 0%.

Results of the evaluation in Comparative Example 3 were a detachment rate of 58.4% and a variation evaluation of D.

(Comparative Example 4)

Comparative Example 4 was carried out under the same conditions as in Example 1, except that the first acoustic transmission medium was not used and the amount of water used as the second acoustic transmission medium was changed.

As the second acoustic transmission medium, water was placed on the vibrating body through use of the dispenser in an amount of 4.0 mL. After the water spread uniformly on the vibrating body, the center of the Nunc (trademark) Φ35-mm dish manufactured by Thermo Fisher Scientific Inc., serving as the culture substrate on which cells were cultured, was slowly placed on the vibrating plate with the centers of both aligned so that no air layer was introduced on the bottom surface of the culture substrate. After that, a weight of 100 g was placed on the lid of the culture substrate to apply pressure. From a photograph taken from above after the application of pressure, it was confirmed that the entire bottom surface of the culture substrate on the vibrating body side was filled with the second acoustic transmission medium and there was no air layer.

The contact area between the first acoustic transmission medium and the culture substrate was 0%.

Results of the evaluation in Comparative Example 4 were a detachment rate of 82.0% and a variation evaluation of D.

(Conclusion)

In Fig. 12, results obtained by summarizing the respective configurations and evaluation results of the above-mentioned Examples and Comparative Examples are shown. The tanδ shown in Fig. 12 indicates the values obtained at room temperature when a vibration having a frequency of 0.1 Hz is applied.

The present invention is not limited to the above-mentioned embodiments, and various changes and modifications are possible without departing from the spirit and scope of the present invention. Accordingly, the claims are attached hereto to make the scope of the present invention public.

The present application claims priority based on Japanese Patent Application No. 2023-118581 filed on July 20, 2023, the entire contents of which are incorporated herein by reference.

[Reference Signs List]

11: vibrating plate
12: piezoelectric body
13: acoustic transmission medium

131: first acoustic transmission medium
132: second acoustic transmission medium
14: weight
15: upper cover
16: cushioning material
17: lower cover
18: bolt
21: culture substrate
22: culture substrate upper lid
23: supply unit
24: arm
30: groove portion
101: vibrating body
205: information processing device
206: CPU
207: RAM
208: ROM
209: HDD
210: communication I/F
211: display device
212: input device

## Claims

1. A cell detachment method of detaching cells adhering to a culture surface of a culture substrate from the culture surface,

   the cell detachment method comprising a detachment step of detaching the cells from the culture surface by vibrating a vibrating body under a state in which a first acoustic transmission medium and a second acoustic transmission medium are arranged between the culture substrate and the vibrating body, and under a state in which at least one of the following (a) or (b) is satisfied:

   (a) the culture substrate is in contact with the first acoustic transmission medium and the second acoustic transmission medium; or
   (b) the vibrating body is in contact with the first acoustic transmission medium and the second acoustic transmission medium,

   wherein the first acoustic transmission medium and the second acoustic transmission medium are different from each other, the first acoustic transmission medium being a solid at room temperature, the second acoustic transmission medium being a liquid or a solid with flowability at room temperature.

2. The cell detachment method according to claim 1, wherein the detachment step includes detaching the cells from the culture surface by vibrating the vibrating body under a state in which the (a) is satisfied.

3. The cell detachment method according to claim 1 or 2, wherein the detachment step includes detaching the cells from the culture surface by vibrating the vibrating body under a state in which the (a) is satisfied, and under a state in which the vibrating body is in contact with the first acoustic transmission medium.

4. The cell detachment method according to claim 2 or 3, wherein the following expression is satisfied in the detachment step: $0.05 \leq S1/S1+S2$, where S1 is an area of a portion in which the culture substrate is in contact with the first acoustic transmission medium, and S2 is an area of a portion in which the culture substrate is in contact with the second acoustic transmission medium.

5. The cell detachment method according to claim 1, wherein the detachment step includes detaching the cells from the culture surface by vibrating the vibrating body under a state in which the (b) is satisfied.

6. The cell detachment method according to claim 5, wherein the following expression is satisfied in the detachment step:

0.05≤S3/S3+S4, where S3 is an area of a portion in which the vibrating body is in contact with the first acoustic transmission medium, and S4 is an area of a portion in which the vibrating body is in contact with the second acoustic transmission medium.

**7.** The cell detachment method according to any one of claims 1 to 6, the following expression is satisfied: $\tan\delta_1<\tan\delta_2$, where $\tan\delta_1$ and $\tan\delta_2$ are a $\tan\delta$ of the first acoustic transmission medium and a $\tan\delta$ of the second acoustic transmission medium, respectively, obtained by dynamic viscoelasticity measurement at room temperature and a frequency of 1 Hz.

**8.** The cell detachment method according to any one of claims 1 to 7, wherein the second acoustic transmission medium is a solid having a $\tan\delta$ of 1.0 or more obtained when a vibration having a frequency of 0.1 Hz is applied at room temperature.

**9.** The cell detachment method according to any one of claims 1 to 8, wherein the second acoustic transmission medium is a liquid at room temperature.

**10.** The cell detachment method according to any one of claims 1 to 9,

wherein the second acoustic transmission medium is a liquid, and
wherein a contact angle between the first acoustic transmission medium and the second acoustic transmission medium is 120° or less.

**11.** The cell detachment method according to claim 10,

wherein the second acoustic transmission medium is a liquid, and
wherein the contact angle between the first acoustic transmission medium and the second acoustic transmission medium is 90° or less.

**12.** The cell detachment method according to any one of claims 1 to 11,

wherein the second acoustic transmission medium is a liquid, and
wherein at least one of a contact angle at a contact surface between the culture substrate and the second acoustic transmission medium or a contact angle at a contact surface between the vibrating body and the second acoustic transmission medium is less than 40°.

**13.** The cell detachment method according to any one of claims 1 to 12, wherein the first acoustic transmission medium includes, in at least one of a surface facing the culture substrate or a surface facing the vibrating body, a continuous groove portion extending from a central portion to an outer peripheral portion of the at least one of the surfaces.

**14.** The cell detachment method according to any one of claims 1 to 13, wherein the detachment step includes setting a volume ratio of the second acoustic transmission medium to the first acoustic transmission medium to 0.05 or more and 2.0 or less.

**15.** The cell detachment method according to any one of claims 1 to 14, wherein the first acoustic transmission medium includes silicone rubber.

**16.** The cell detachment method according to any one of claims 1 to 15, wherein the first acoustic transmission medium includes a porous resin material.

**17.** The cell detachment method according to any one of claims 1 to 16, wherein the second acoustic transmission medium includes at least one type selected from the group consisting of water, physiological saline, ethanol, and diluted ethanol.

**18.** The cell detachment method according to any one of claims 1 to 17, wherein the detachment step includes a step of exciting vibration in an ultrasonic band in the vibrating body.

**19.** The cell detachment method according to any one of claims 1 to 18, further comprising an arrangement step of arranging the first acoustic transmission medium and the second acoustic transmission medium between the culture substrate and the vibrating body before the detachment step.

**20.** The cell detachment method according to claim 19, wherein the arrangement step includes arranging any one of the first acoustic transmission medium or the second acoustic transmission medium so as to be in contact with any one of the vibrating body or the culture substrate based on a state of vibration to be excited in the vibrating body in the detachment step.

**21.** The cell detachment method according to claim 19 or 20, wherein the arrangement step includes supplying the second acoustic transmission medium by supply unit including at least one selected from the group consisting of a dispenser, a nozzle, a spray, and a sponge, and arranging the second acoustic transmission medium so as to be in contact with the first acoustic transmission medium.

**22.** The cell detachment method according to any one of claims 1 to 21, further comprising a pressing step of applying pressure so as to bring the culture substrate and the vibrating body closer to each other.

**23.** The cell detachment method according to any one of claims 1 to 22, further comprising a surface treatment step of applying surface treatment to at least one surface selected from the group consisting of:

a surface of the culture substrate facing the first acoustic transmission medium;
a surface of the vibrating body facing the first acoustic transmission medium;
a surface of the first acoustic transmission medium facing the culture substrate; and
a surface of the first acoustic transmission medium facing the vibrating body.

**24.** A cell detachment device for detaching cells from a culture surface of a culture substrate by transmitting vibration generated by a vibrating body to the cells provided on the culture surface, the cell detachment device comprising:

a vibrating body;
a first acoustic transmission medium; and
a culture substrate placement portion, in the stated order, and
the cell detachment device further comprising supply unit for supplying a second acoustic transmission medium, wherein the supply unit is configured to supply the second acoustic transmission medium so that at least one of the following (a) or (b) is satisfied:

(a) the culture substrate is in contact with the first acoustic transmission medium and the second acoustic transmission medium; or
(b) the vibrating body is in contact with the first acoustic transmission medium and the second acoustic transmission medium.

**25.** The cell detachment device according to claim 24, further comprising an information processing device, wherein the information processing device is configured to:

acquire information regarding a state of vibration to be excited in the vibrating body; and
control the supply unit based on the information.

**26.** The cell detachment device according to claim 24 or 25, wherein the vibrating body includes an ultrasonic vibrator.

**27.** The cell detachment device according to any one of claims 24 to 26, comprising pressing unit for applying pressure so as to bring the culture substrate and the vibrating body closer to each other.

FIG. 1A

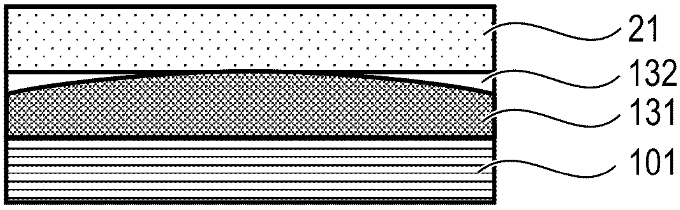


FIG. 1B

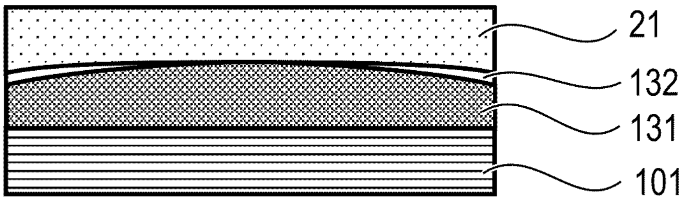


FIG. 1C

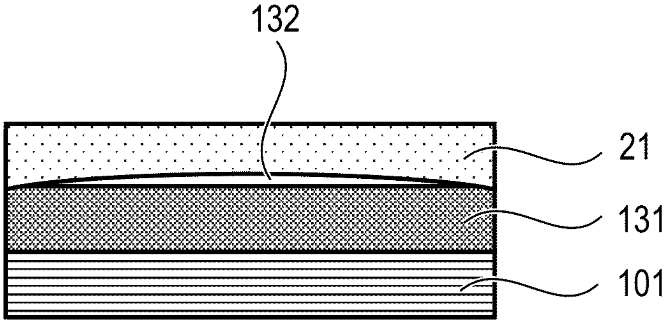

FIG. 2A

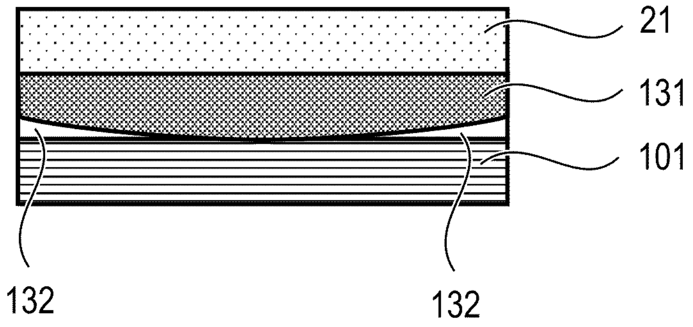


FIG. 2B

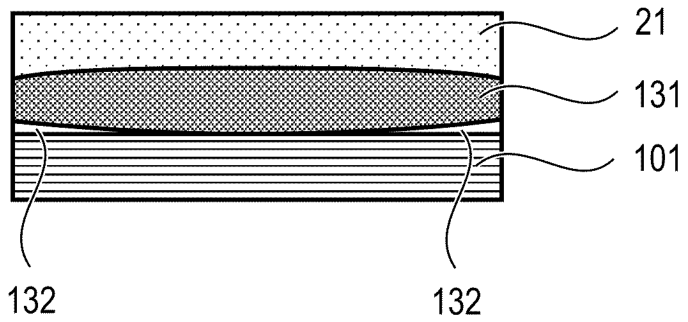


FIG. 2C

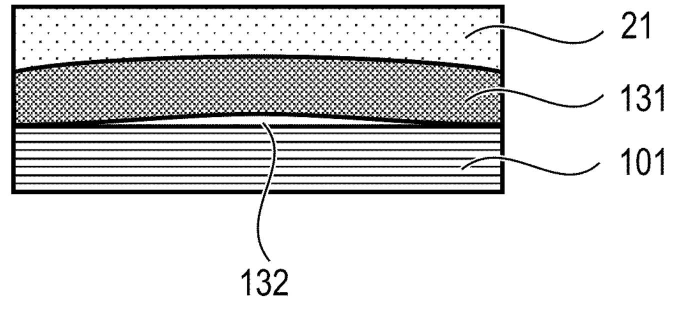

FIG. 3A

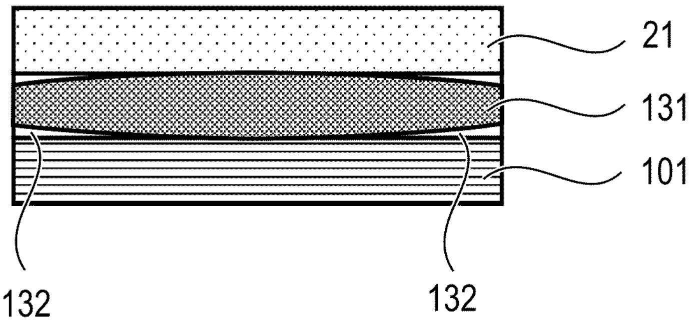


FIG. 3B

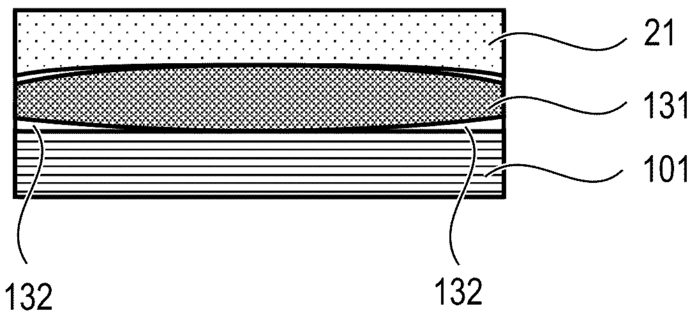


FIG. 3C

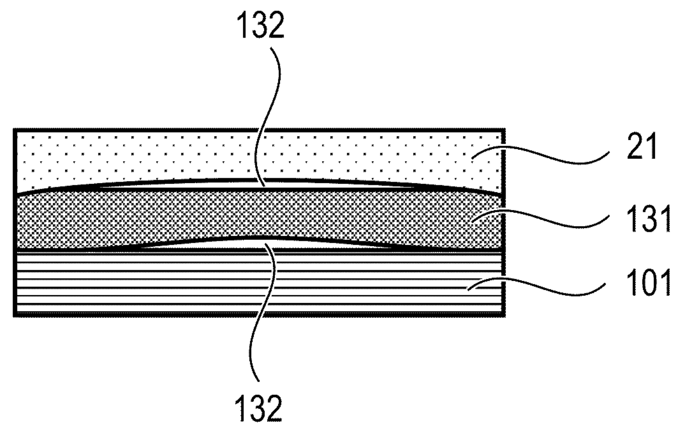

FIG. 4A

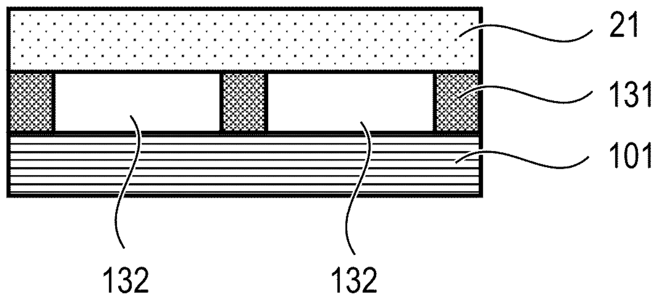


FIG. 4B

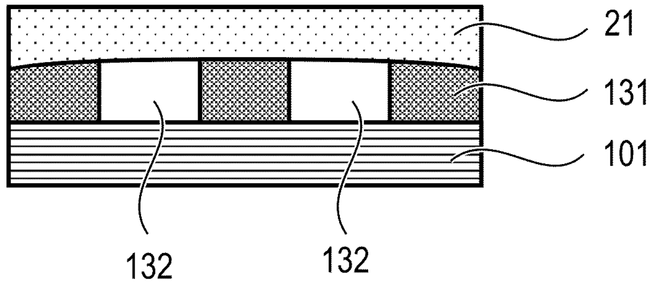


FIG. 4C

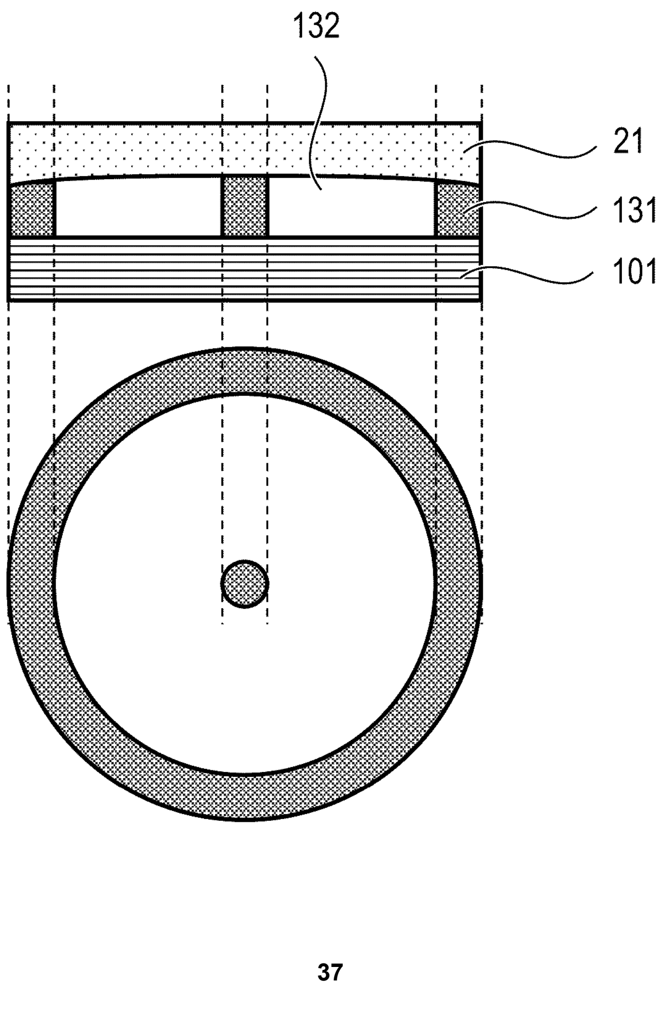

FIG. 5A

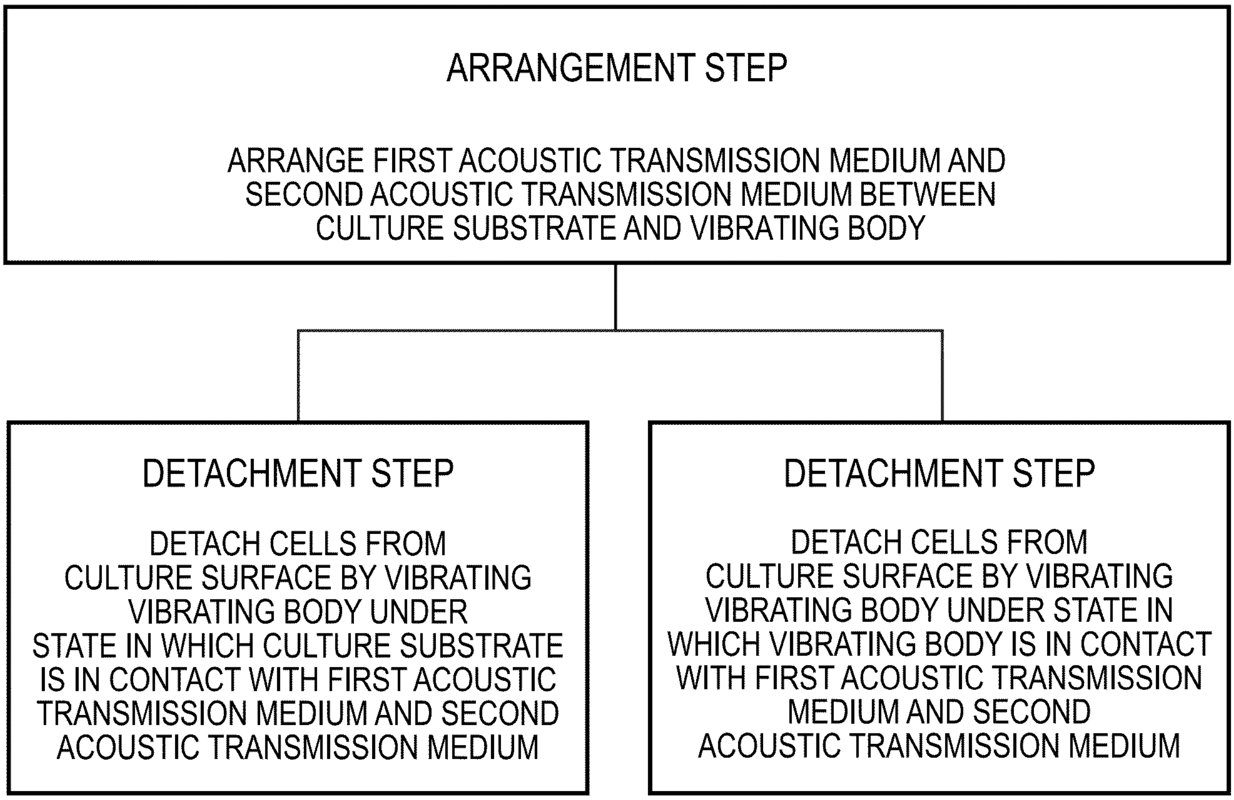
ARRANGEMENT STEP
ARRANGE FIRST ACOUSTIC TRANSMISSION MEDIUM AND SECOND ACOUSTIC TRANSMISSION MEDIUM BETWEEN CULTURE SUBSTRATE AND VIBRATING BODY
DETACHMENT STEP
DETACH CELLS FROM CULTURE SURFACE BY VIBRATING VIBRATING BODY UNDER STATE IN WHICH CULTURE SUBSTRATE IS IN CONTACT WITH FIRST ACOUSTIC TRANSMISSION MEDIUM AND SECOND ACOUSTIC TRANSMISSION MEDIUM
DETACHMENT STEP
DETACH CELLS FROM CULTURE SURFACE BY VIBRATING VIBRATING BODY UNDER STATE IN WHICH VIBRATING BODY IS IN CONTACT WITH FIRST ACOUSTIC TRANSMISSION MEDIUM AND SECOND ACOUSTIC TRANSMISSION MEDIUM

FIG. 5B

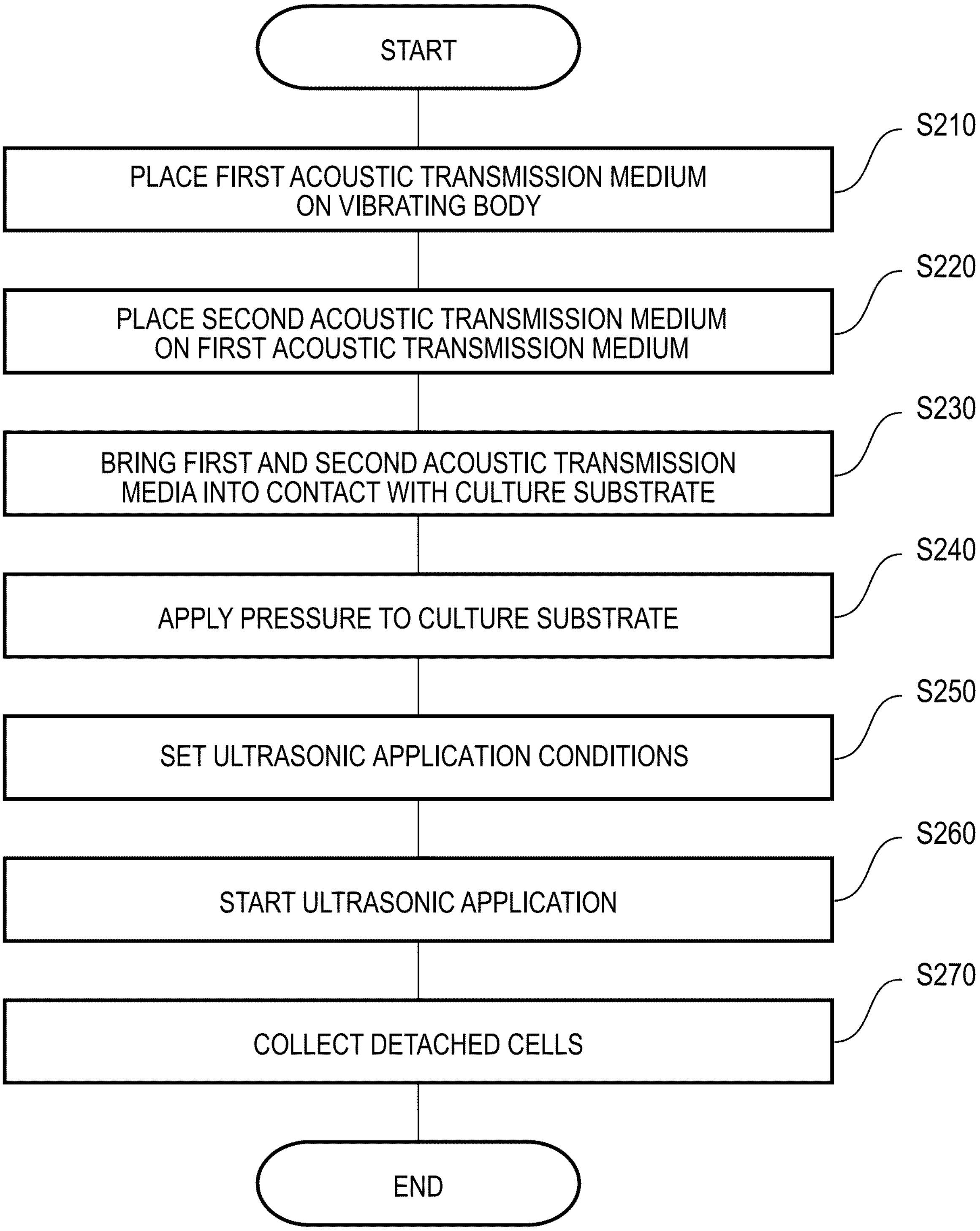
START
S210
PLACE FIRST ACOUSTIC TRANSMISSION MEDIUM ON VIBRATING BODY
S220
PLACE SECOND ACOUSTIC TRANSMISSION MEDIUM ON FIRST ACOUSTIC TRANSMISSION MEDIUM
S230
BRING FIRST AND SECOND ACOUSTIC TRANSMISSION MEDIA INTO CONTACT WITH CULTURE SUBSTRATE
S240
APPLY PRESSURE TO CULTURE SUBSTRATE
S250
SET ULTRASONIC APPLICATION CONDITIONS
S260
START ULTRASONIC APPLICATION
S270
COLLECT DETACHED CELLS
END

FIG. 6A

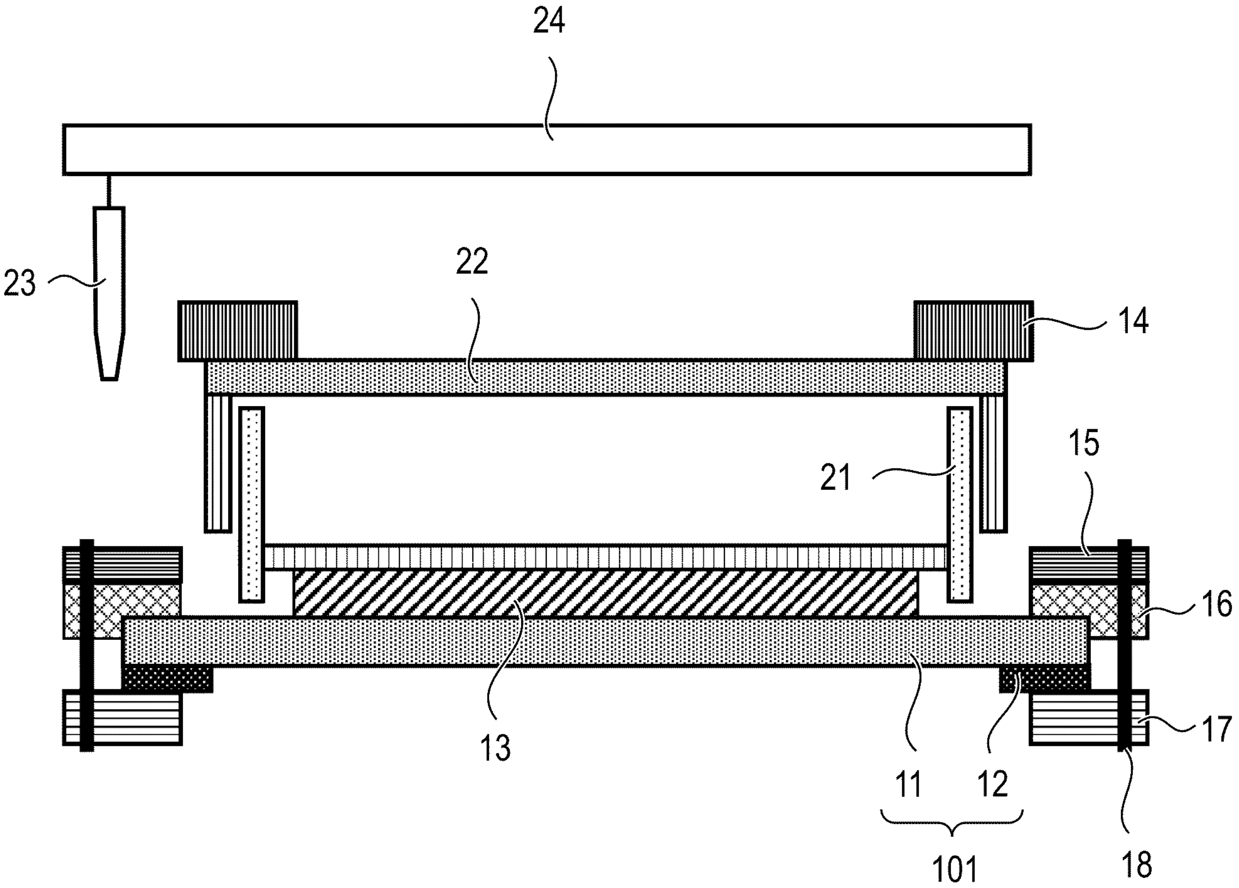

FIG. 6B

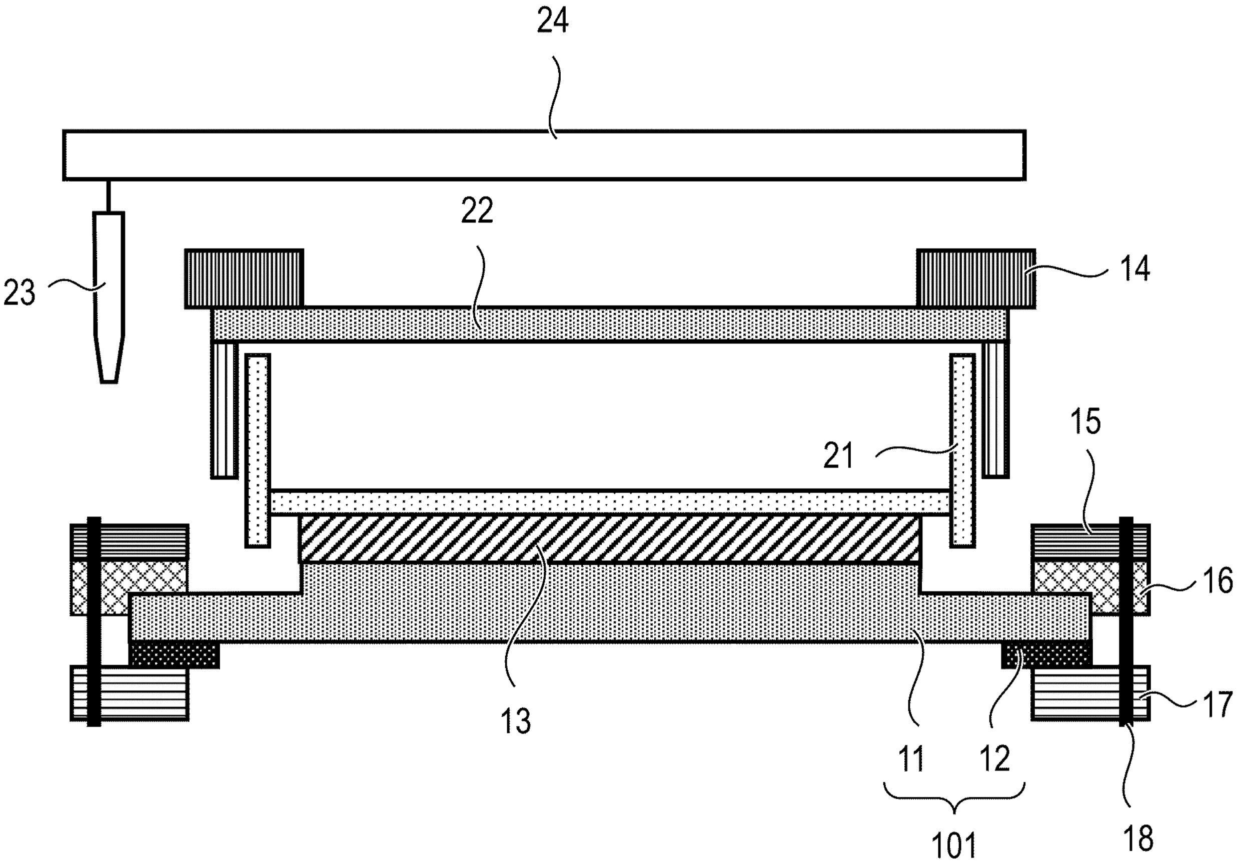

FIG. 6C

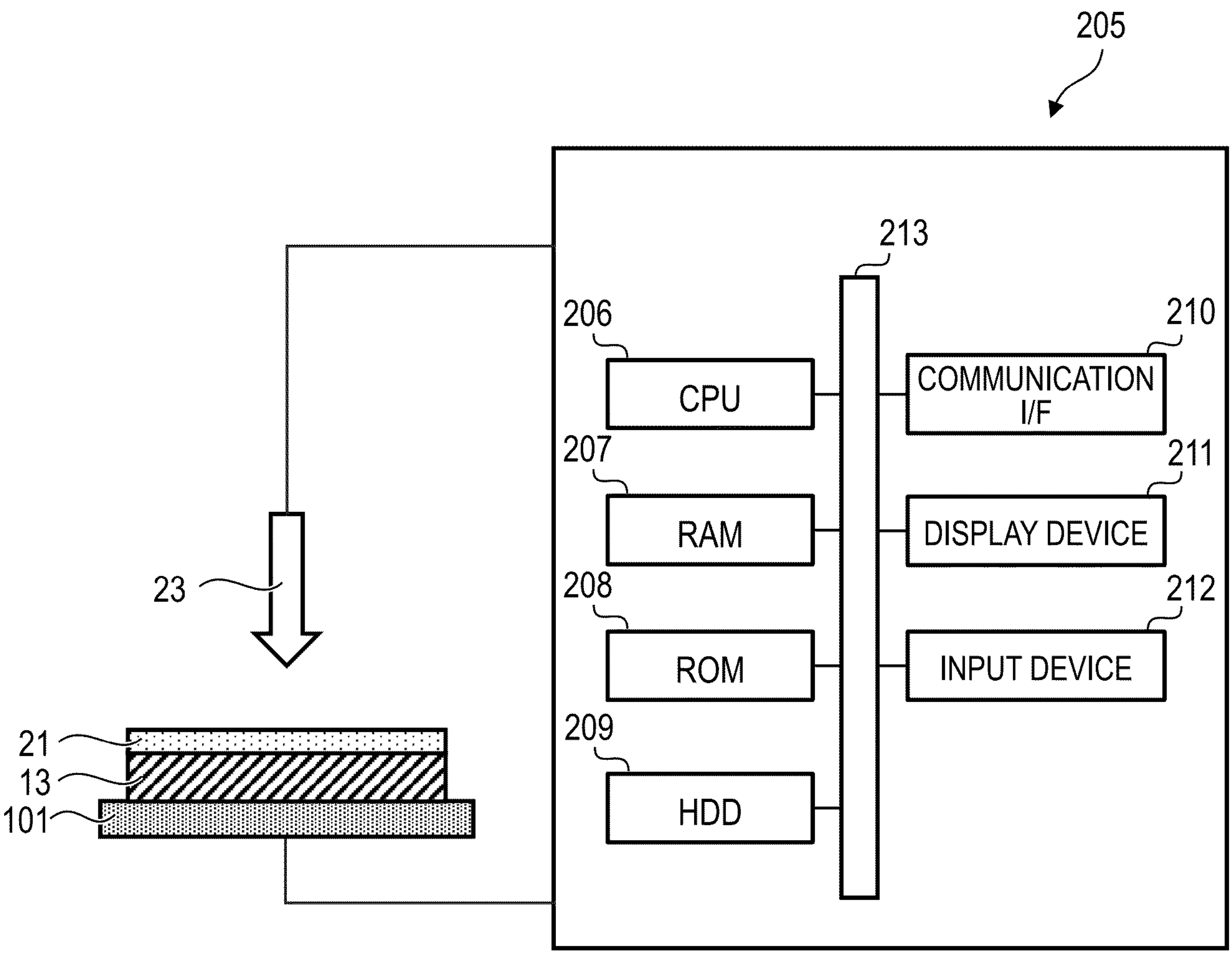
205
213
206
CPU
207
RAM
208
ROM
209
HDD
210
COMMUNICATION I/F
211
DISPLAY DEVICE
212
INPUT DEVICE
23
21
13
101

FIG. 7A

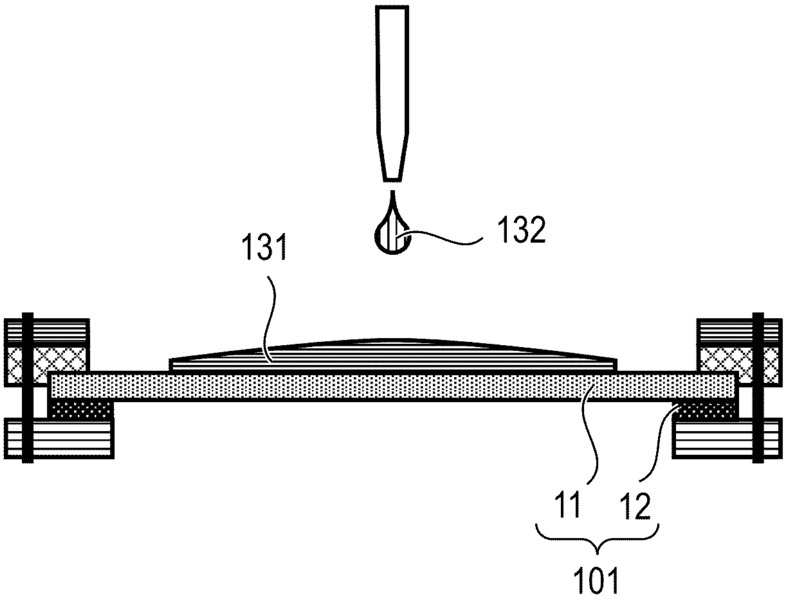


FIG. 7B

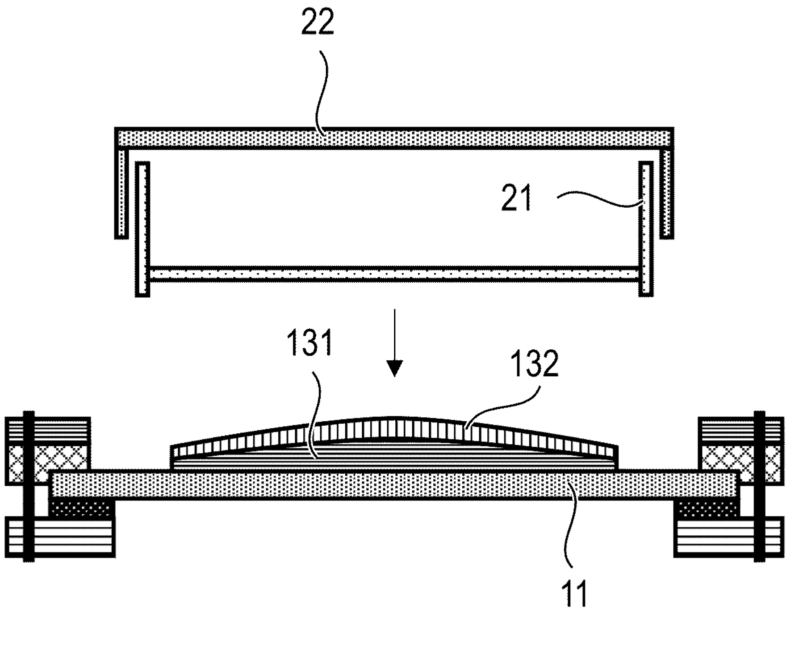

FIG. 7C

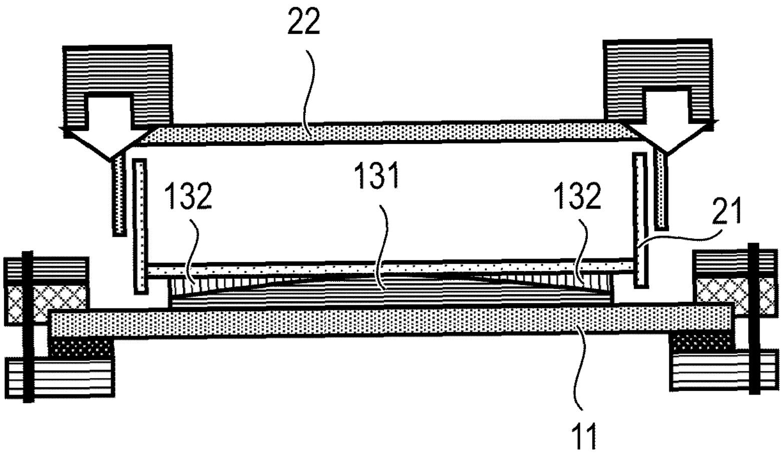


FIG. 7D

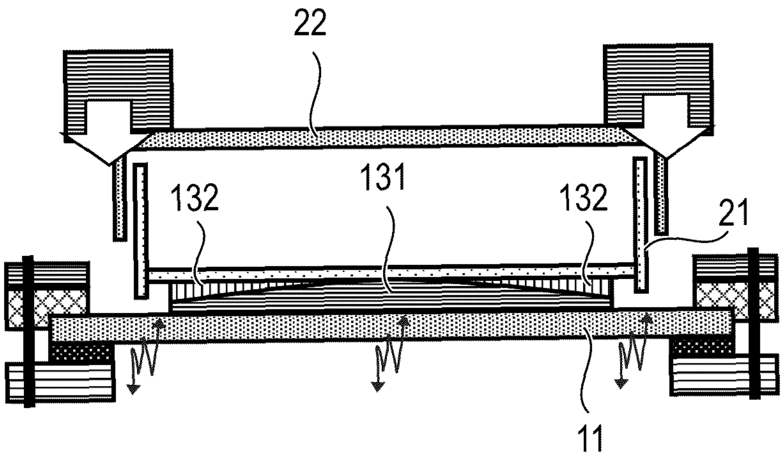

FIG. 8

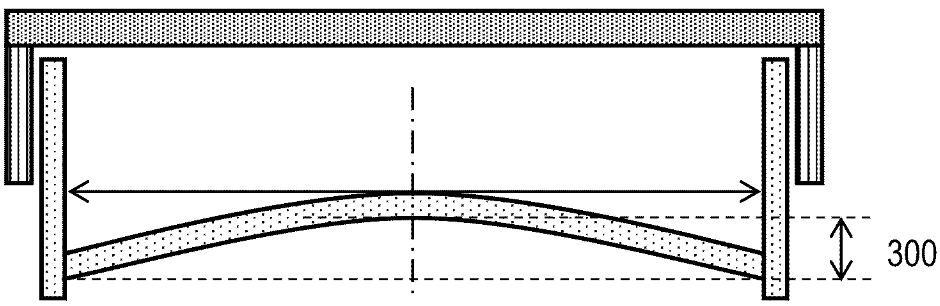

FIG. 9A

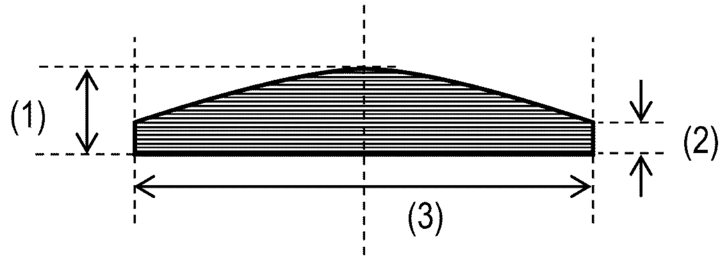


FIG. 9B

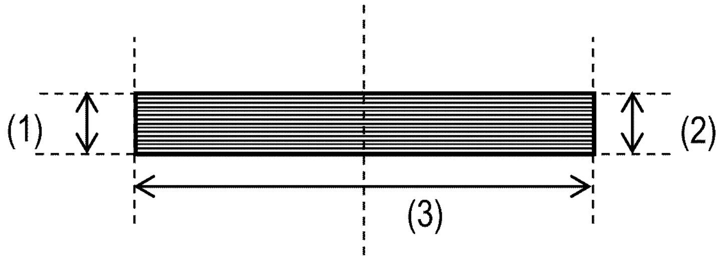


FIG. 9C

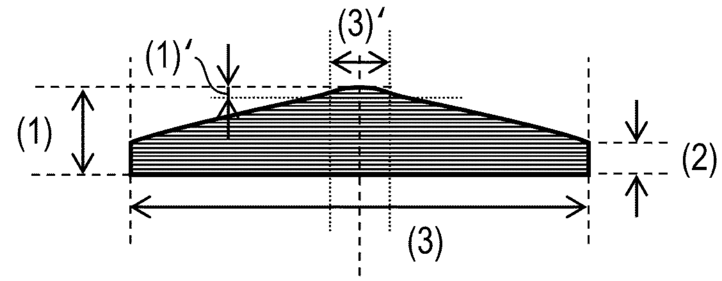

FIG. 9D

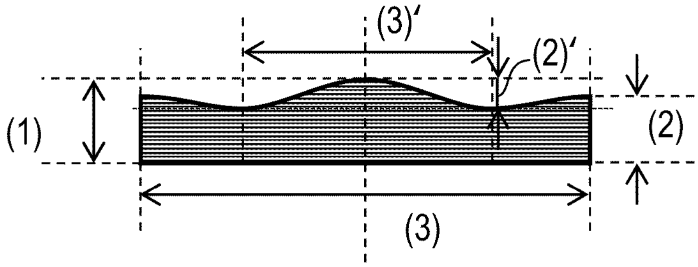


FIG. 9E

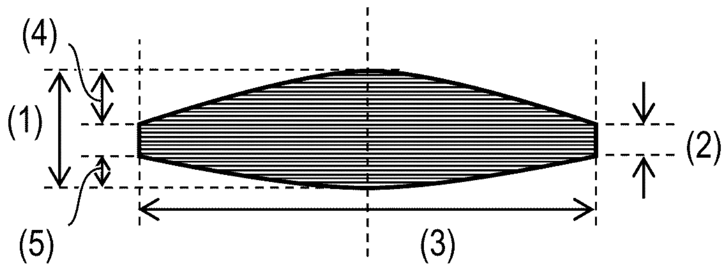


FIG. 9F

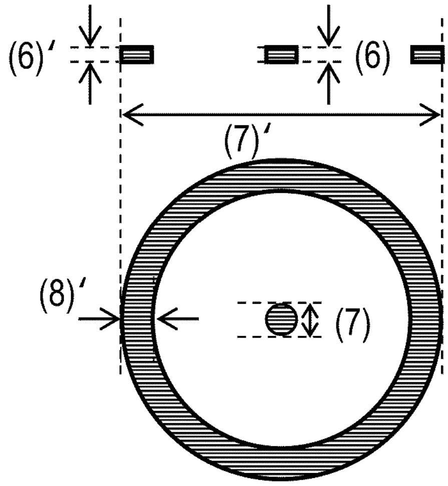

FIG. 10

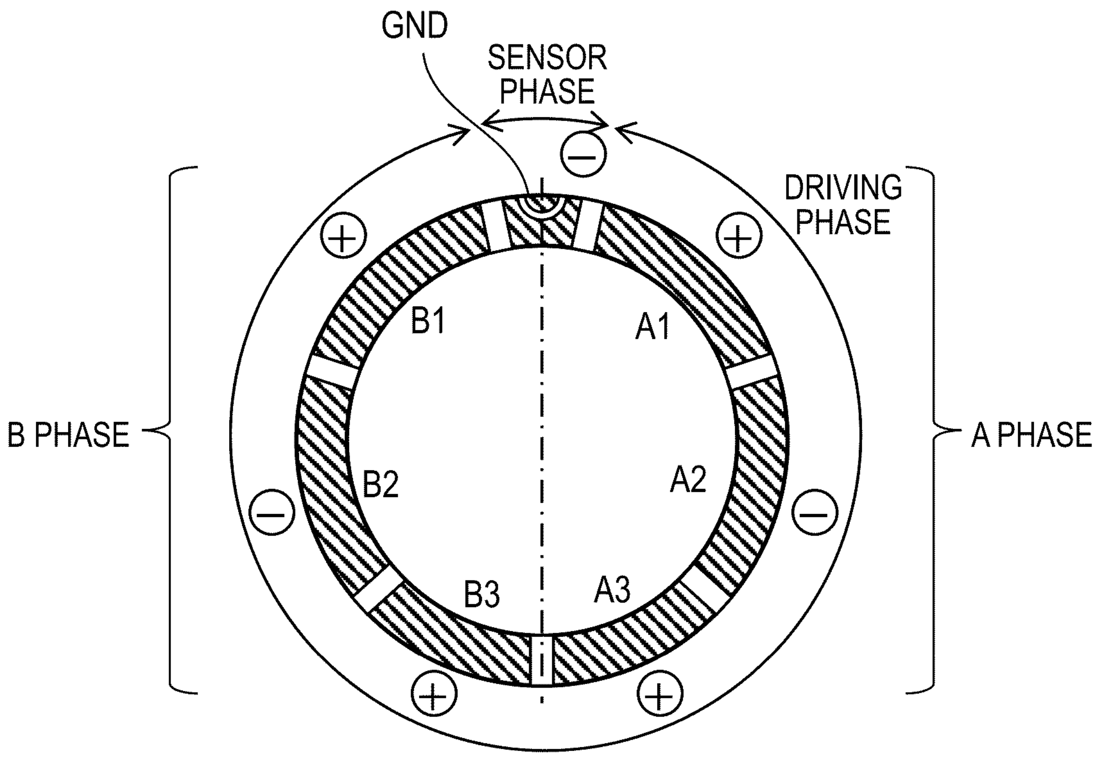
GND
SENSOR PHASE
DRIVING PHASE
B PHASE
A PHASE
B1
B2
B3
A1
A2
A3

FIG. 11

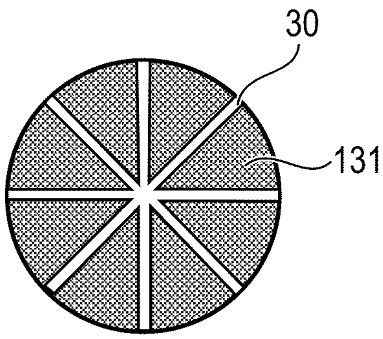

# FIG. 12

| | ACOUSTIC TRANSMISSION MEDIUM | | | | | | | | VIBRATION MODE | DETACHMENT RATE % | VARIATION |
|---|---|---|---|---|---|---|---|---|---|---|---|
| | FIRST ACOUSTIC TRANSMISSION MEDIUM | | | SECOND ACOUSTIC TRANSMISSION MEDIUM | | | CONTACT ANGLE BETWEEN FIRST ACOUSTIC TRANSMISSION MEDIUM AND SECOND ACOUSTIC TRANSMISSION MEDIUM | VOLUME RATIO (VOLUME OF SECOND ACOUSTIC TRANSMISSION MEDIUM / VOLUME OF FIRST ACOUSTIC TRANSMISSION MEDIUM) | | | |
| | MATERIAL | CONTACT AREA RATIO OF FIRST ACOUSTIC TRANSMISSION | tanδ | MATERIAL | VISCOSITY | tanδ | | | | | |
| EXAMPLE 1 | SILICONE RUBBER | 10% | 0.13 | WATER | 0.9 | LIQUID | 110 | 0.31 | A | 97.4 | A |
| EXAMPLE 2 | SILICONE RUBBER | 20% | 0.13 | WATER | 0.9 | LIQUID | 110 | 0.1 | A | 90.3 | C |
| EXAMPLE 3 | SILICONE RUBBER | 10% | 0.13 | WATER | 0.9 | LIQUID | 110 | 0.31 | B | 90 | C |
| EXAMPLE 4 | SILICONE RUBBER | 20% | 0.13 | WATER | 0.9 | LIQUID | 110 | 0.1 | B | 96.8 | A |
| EXAMPLE 5 | SILICONE RUBBER | 5% | 0.13 | WATER | 0.9 | LIQUID | 110 | 0.45 | A | 90.2 | B |
| EXAMPLE 6 | SILICONE RUBBER | 30% | 0.13 | WATER | 0.9 | LIQUID | 110 | 0.35 | A | 98.2 | A |
| EXAMPLE 7 | SILICONE RUBBER | 10% | 0.13 | PVA GEL | 1.1×10^6 | 0.856 | - | 0.34 | A | 85.7 | C |
| EXAMPLE 8 | SILICONE RUBBER | 10% | 0.13 | ULTRASOUND GEL | 6.6×10^4 | LIQUID | - | 0.34 | A | 96.4 | A |
| EXAMPLE 9 | SILICONE RUBBER | 10% | 0.13 | GLYCERIN | 850 | LIQUID | 100 | 0.34 | A | 94.3 | A |
| EXAMPLE 10 | SILICONE RUBBER | 10% | 0.13 | WATER | 0.9 | LIQUID | 110 | 2.2 | A | 92.1 | A |
| EXAMPLE 11 | SILICONE RUBBER | 10% | 0.13 | ETHANOL | 1.1 | LIQUID | 40 | 2.2 | A | 94 | A |
| EXAMPLE 12 | SILICONE RUBBER | 10% | 0.13 | 70% ETHANOL AQUEOUS SOLUTION | 2.1 | LIQUID | 55 | 2.2 | A | 94.6 | A |
| EXAMPLE 13 | SILICONE RUBBER | 10% | 0.13 | WATER + SURFACTANT 1 | 0.9 | LIQUID | 50 | 2.2 | A | 93.9 | A |
| EXAMPLE 14 | SILICONE RUBBER | 10% | 0.13 | WATER + SURFACTANT 2 | 0.9 | LIQUID | 75 | 2.2 | A | 93.2 | A |
| EXAMPLE 15 | SILICONE RUBBER | 10% | 0.13 | WATER | 0.9 | LIQUID | 25 | 2.2 | A | 95.5 | A |
| EXAMPLE 16 | SILICONE RUBBER | 10% | 0.13 | WATER | 0.9 | LIQUID | 110 | 2.2 | A | 95.1 | A |

FIG. 12
(continued 1)

| | ACOUSTIC TRANSMISSION MEDIUM | | | | | | | | VIBRATION MODE | DETACH-MENT RATE % | VARIATION |
|---|---|---|---|---|---|---|---|---|---|---|---|
| | FIRST ACOUSTIC TRANSMISSION MEDIUM | | | SECOND ACOUSTIC TRANSMISSION MEDIUM | | | CONTACT ANGLE BETWEEN FIRST ACOUSTIC TRANSMISSION MEDIUM AND SECOND ACOUSTIC TRANSMISSION MEDIUM | VOLUME RATIO (VOLUME OF SECOND ACOUSTIC TRANSMISSION MEDIUM / VOLUME OF FIRST ACOUSTIC TRANSMISSION MEDIUM) | | | |
| | MATERIAL | CONTACT AREA RATIO OF FIRST ACOUSTIC TRANSMISSION | tanδ | MATERIAL | VISCOS-ITY | tanδ | | | | | |
| EXAMPLE 17 | SILICONE RUBBER | 10% | 0.13 | WATER | 0.9 | LIQUID | 110 | 2.2 | A | 94.8 | A |
| EXAMPLE 18 | SILICONE RUBBER | 10% | 0.13 | WATER | 0.9 | LIQUID | 110 | 2.2 | A | 95.8 | A |
| EXAMPLE 19 | SILICONE RUBBER | 10% | 0.13 | 70% ETHANOL AQUEOUS SOLUTION | 0.9 | LIQUID | 55 | 2.2 | A | 97.4 | A |
| EXAMPLE 20 | SILICONE RUBBER | 10% | 0.13 | 70% ETHANOL AQUEOUS SOLUTION | 2.1 | LIQUID | 55 | 2.2 | A | 97.1 | A |
| EXAMPLE 21 | SILICONE RUBBER | 10% | 0.13 | WATER | 0.9 | LIQUID | 110 | 1.9 | A | 91.1 | B |
| EXAMPLE 22 | SILICONE RUBBER | 20% | 0.13 | WATER | 0.9 | LIQUID | 110 | 0.054 | A | 90.2 | C |
| EXAMPLE 23 | SILICONE RUBBER | 10% | 0.13 | WATER | 0.9 | LIQUID | 110 | 2.2 | A | 90.4 | B |
| EXAMPLE 24 | SILICONE RUBBER | 20% | 0.13 | WATER | 0.9 | LIQUID | 110 | 0.047 | A | 84.2 | C |
| EXAMPLE 25 | SILICONE RUBBER | 5% | 0.13 | WATER | 0.9 | LIQUID | 110 | 0.045 | A | 83.5 | C |
| EXAMPLE 26 | URETHANE RUBBER | 10% | 0.23 | WATER | 0.9 | LIQUID | 80 | 0.31 | A | 90.1 | B |
| EXAMPLE 27 | SILICONE RUBBER | 15% | 0.14 | WATER | 0.9 | LIQUID | 110 | 0.31 | A | 93.3 | B |
| EXAMPLE 28 | SILICONE RUBBER | 5% | 0.05 | WATER | 0.9 | LIQUID | 110 | 0.31 | A | 96.4 | A |
| EXAMPLE 29 | SILICONE RUBBER | 10% | 0.13 | WATER | 0.9 | LIQUID | 110 | 0.31 | A | 97.2 | A |
| EXAMPLE 30 | SILICONE RUBBER | 10% | 0.13 | WATER | 0.9 | LIQUID | 110 | 0.45 | A | 94.4 | B |

# FIG. 12 (continued 2)

| | ACOUSTIC TRANSMISSION MEDIUM | | | | | | | | VIBRATION MODE | DETACHMENT RATE % | VARIATION |
|---|---|---|---|---|---|---|---|---|---|---|---|
| | FIRST ACOUSTIC TRANSMISSION MEDIUM | | | SECOND ACOUSTIC TRANSMISSION MEDIUM | | | CONTACT ANGLE BETWEEN FIRST ACOUSTIC TRANSMISSION MEDIUM AND SECOND ACOUSTIC TRANSMISSION MEDIUM | VOLUME RATIO (VOLUME OF SECOND ACOUSTIC TRANSMISSION MEDIUM / VOLUME OF FIRST ACOUSTIC TRANSMISSION MEDIUM) | | | |
| | MATERIAL | CONTACT AREA RATIO OF FIRST ACOUSTIC TRANSMISSION | tanδ | MATERIAL | VISCOSITY | tanδ | | | | | |
| EXAMPLE 31 | SILICONE RUBBER | 10% | 0.13 | WATER | 0.9 | LIQUID | 110 | 1.74 | A | 92.3 | C |
| EXAMPLE 32 | SILICONE RUBBER | 10% | 0.13 | WATER | 0.9 | LIQUID | 110 | 0.31 | C | 99.4 | A |
| EXAMPLE 33 | SILICONE RUBBER | 3% | 0.13 | WATER | 0.9 | LIQUID | 110 | 0.25 | A | 84.1 | C |
| COMPARATIVE EXAMPLE 1 | SILICONE RUBBER | 5% | 0.13 | - | - | - | - | - | A | 81.2 | D |
| COMPARATIVE EXAMPLE 2 | SILICONE RUBBER | 5% | 0.13 | - | - | - | - | - | B | 70.8 | D |
| COMPARATIVE EXAMPLE 3 | SILICONE RUBBER | 0% | 0.13 | WATER | 0.9 | LIQUID | 110 | 0.47 | A | 58.4 | D |
| COMPARATIVE EXAMPLE 4 | - | 0% | - | WATER | 0.9 | LIQUID | - | - | A | 82 | D |

**INTERNATIONAL SEARCH REPORT**

International application No.
**PCT/JP2024/025525**

**A. CLASSIFICATION OF SUBJECT MATTER**

***C12M 3/00***(2006.01)i; ***C12N 5/071***(2010.01)i
FI: C12M3/00 Z; C12N5/071

According to International Patent Classification (IPC) or to both national classification and IPC

**B. FIELDS SEARCHED**

Minimum documentation searched (classification system followed by classification symbols)

C12M3/00; C12N5/071

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

JSTPlus/JMEDPlus/JST7580 (JDreamIII)

**C. DOCUMENTS CONSIDERED TO BE RELEVANT**

| Category* | Citation of document, with indication, where appropriate, of the relevant passages | Relevant to claim No. |
|---|---|---|
| A | WO 2019/044804 A1 (KEIO UNIVERSITY) 07 March 2019 (2019-03-07)<br>claims, paragraphs [0020], [0045], [0050], fig. 1-4 | 1-27 |
| A | JP 2017-46591 A (TOKYO ELECTRON LIMITED) 09 March 2017 (2017-03-09)<br>claims, paragraphs [0068], [0069] | 1-27 |
| A | IMASHIRO, Chikahiro. Detachment of cell sheets from clinically ubiquitous cell culture vessels by ultrasonic vibration. Scientific Reports. 2020, https://doi.org/10.1038/s41598-020-66375-1, Results, fig. 1<br>Results, fig. 1 | 1-27 |
| P, A | JP 2024-66306 A (CANON KABUSHIKI KAISHA) 15 May 2024 (2024-05-15)<br>claims, paragraph [0043], drawings | 1-27 |

☐ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **17 September 2024** | **01 October 2024** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (July 2022)

**INTERNATIONAL SEARCH REPORT**
**Information on patent family members**

International application No.
**PCT/JP2024/025525**

| Patent document cited in search report | Publication date (day/month/year) | Patent family member(s) | Publication date (day/month/year) |
|---|---|---|---|
| WO 2019/044804 A1 | 07 March 2019 | (Family: none) | |
| JP 2017-46591 A | 09 March 2017 | (Family: none) | |
| JP 2024-66306 A | 15 May 2024 | (Family: none) | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2018042534 A
- JP 2023118581 A